# EUROPÄISCHE PATENTSCHRIFT

(11) **EP 3 744 155 B1**
(45) Veröffentlichungstag und Bekanntmachung des Hinweises auf die Patenterteilung: **02.11.2022**
(21) Anmeldenummer: 19701350.1
(22) Anmeldetag: 23.01.2019
(51) Int. Cl.: H05B 33/20, H01L 51/50, C07D 487/14, C07D 487/22

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 25.01.2018 EP 18153359
(43) Veröffentlichungstag der Anmeldung: 02.12.2020
(73) Patentinhaber: Merck Patent GmbH, 64293 Darmstadt (DE)
(72) Erfinder: PARHAM, Amir, 60486 FRANKFURT AM MAIN (DE); KROEBER, Jonas, 60311 FRANKFURT AM MAIN (DE); ENGELHART, Jens, 64285 DARMSTADT (DE); JATSCH, Anja, 60489 FRANKFURT AM MAIN (DE); EICKHOFF, Christian, 68259 MANNHEIM (DE); EHRENREICH, Christian, 64285 DARMSTADT (DE); JOOSTEN, Dominik, 64372 OBER-RAMSTADT (DE)
(86) Internationale Anmeldenummer: PCT/EP2019/051549
(87) Internationale Veröffentlichungsnummer: WO 2019/145316

(56) Entgegenhaltungen:
- WO-A1-2015/099486
- US-A1- 2014 225 046
- US-A1- 2015 318 478
- US-A1- 2016 233 442

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese Materialien.

In organischen Elektrolumineszenzvorrichtungen (OLEDs) werden als emittierende Materialien häufig phosphoreszierende metallorganische Komplexe eingesetzt. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu deutlichen Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter, und dort zu verbesserten Eigenschaften führen, insbesondere zu einer verbesserten Lebensdauer.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen, insbesondere in Bezug auf die Lebensdauer. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus WO 2010/136109 sind Indenocarbazol-Derivate als Matrixmaterialien für phosphoreszierende Emitter bekannt. Verbindungen gemäß der vorliegenden Erfindung sind nicht offenbart.

Aus US 2015/0318478 ist ein Indenocarbazol-Derivat bekannt, welches am Stickstoffatom mit einer meta-Phenylengruppe substituiert ist, welche wiederum mit einer Diphenyltriazingruppe substituiert ist. Verbindungen mit einer ankondensierten Benzogruppe am Indenocarbazol sind nicht offenbart.

Aus WO 2015/099486 sind Benzoindenocarbazol-Derivate bekannt, welche am Stickstoffatom mit einer meta-Phenylengruppe substituiert sind, welche wiederum mit einer Phenylchinazolingruppe bzw. einer Phenylchinoxalingruppe substituiert ist. Verbindungen, die mit anderen heteroaromatischen Gruppen substituiert sind, sind nicht offenbart.

Gegenstand der vorliegenden Erfindung ist eine Verbindung gemäß Formel (1), 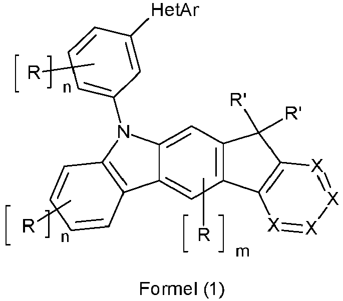 wobei für die verwendeten Symbole und Indizes gilt:
- X: zwei benachbarte X stehen für eine Gruppe der folgenden Formel (2), und die beiden anderen X stehen für CR, 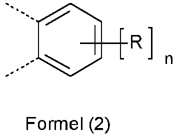 wobei die beiden gestrichelten Bindungen die Verknüpfung dieser Gruppe darstellen;
- HetAr: ist eine Gruppe gemäß einer der Formeln (HetAr-1), (HetAr-3), (HetAr-4) oder (HetAr-5), 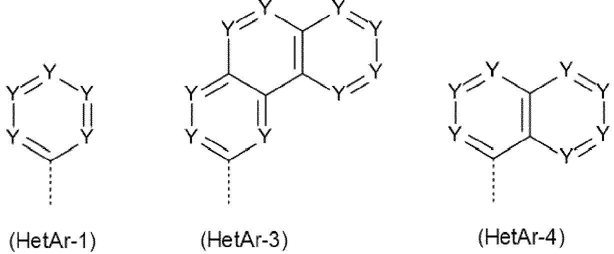 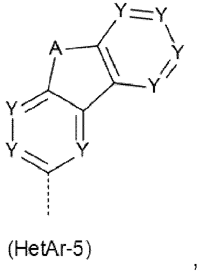 wobei die gestrichelte Bindung die Bindung an die Phenylengruppe darstellt;
- Y: ist gleich oder verschieden bei jedem Auftreten CR oder N, mit der Maßgabe, dass mindestens ein Symbol Y für N steht und dass maximal drei Symbole Y für N stehen;
- A: ist C(R¹)₂, NR¹, O oder S;
- R: ist bei jedem Auftreten gleich oder verschieden H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
- R': ist bei jedem Auftreten gleich oder verschieden eine geradkettige Alkylgruppe mit 1 bis 20 C-Atomen oder eine verzweigte oder cyclische Alkylgruppe mit 3 bis 20 C-Atomen, wobei die geradkettige, verzweigte oder cyclische Alkylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R' auch miteinander ein aromatisches, heteroaromatisches, aliphatisches oder heteroaliphatisches Ringsystem bilden;
- Ar': ist bei jedem Auftreten gleich oder verschieden ein aromatisches oder heteroaromatisches Ringsystem mit 5 bis 40 aromatischen Ringatomen, welches mit einem oder mehreren Resten R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
- R²: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- m: ist 0, 1 oder 2;
- n: ist bei jedem Auftreten gleich oder verschieden 0, 1, 2, 3 oder 4.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Wenn zwei Reste R bzw. R' bzw. R¹ miteinander ein Ringsystem bilden, so kann dieses mono- oder polycyclisch sein. Dabei sind die Reste, die miteinander ein Ringsystem bilden, bevorzugt benachbart, d. h. dass diese Reste an dasselbe Kohlenstoffatom oder an Kohlenstoffatome gebunden sind, die direkt aneinander gebunden sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

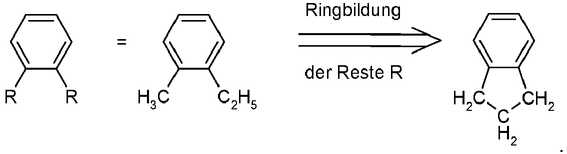

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

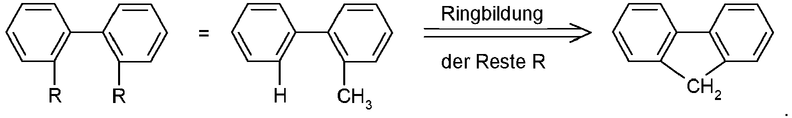

Je nach Position, in der die Gruppe der Formel (2) ankondensiert ist, umfasst die Erfindung die Verbindungen der folgenden Formeln (3), (4) und (5), 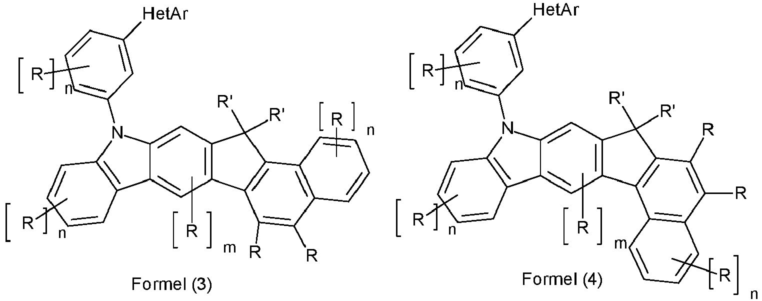 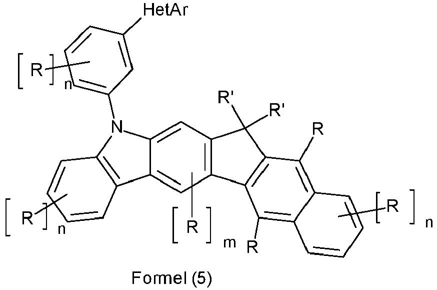 wobei die verwendeten Symbole und Indizes die oben genannten Bedeutungen aufweisen.

In einer bevorzugten Ausführungsform der Erfindung sind die Verbindungen der Formeln (3), (4) und (5) gewählt aus den Verbindungen der folgenden Formeln (3a-1), (3a-2), (4a-1), (4a-2), (5a-1) und (5a-2), 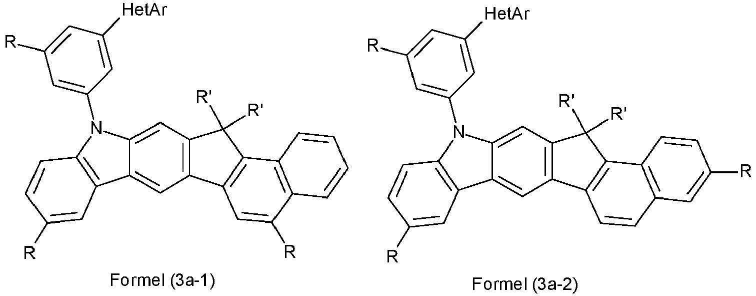 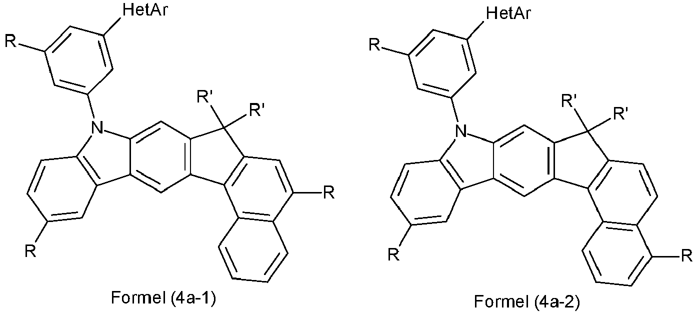 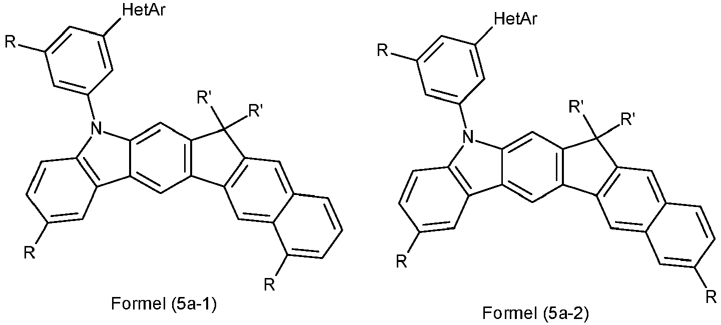 wobei HetAr, R und R' die oben genannten Bedeutungen aufweisen.

Besonders bevorzugt sind die Verbindungen der Formeln (3), (4) und (5) ausgewählt aus den Verbindungen der folgenden Formeln (3b), (4b) und (5b), 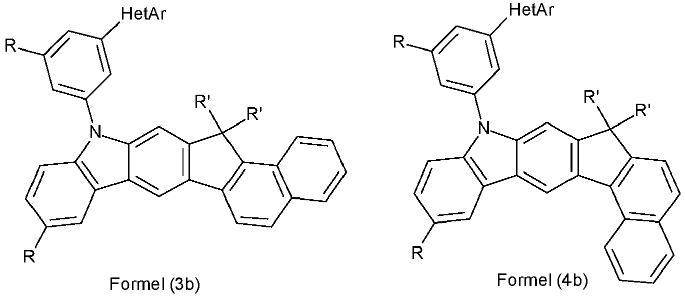 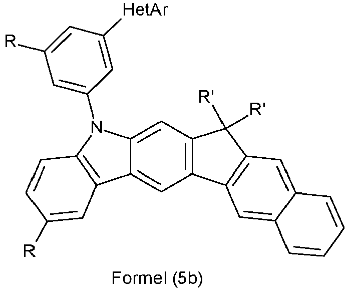 wobei HetAr, R und R' die oben genannten Bedeutungen aufweisen.

Ganz besonders bevorzugt sind die Verbindungen der Formeln (3), (4) und (5) gewählt aus den Verbindungen der folgenden Formeln (3c-1), (3c-2), (4c-1), (4c-2), (5c-1) und (5c-2), 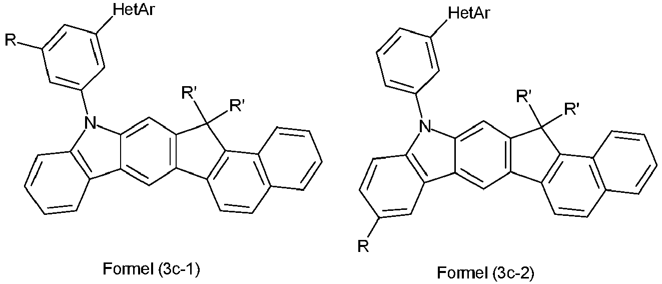 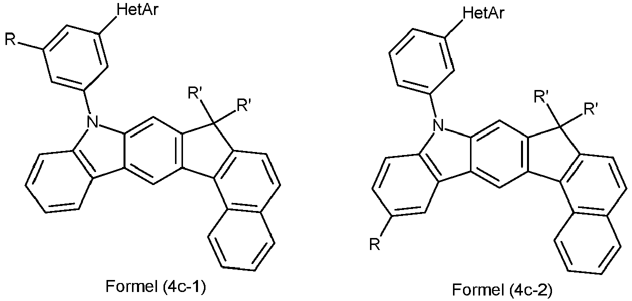 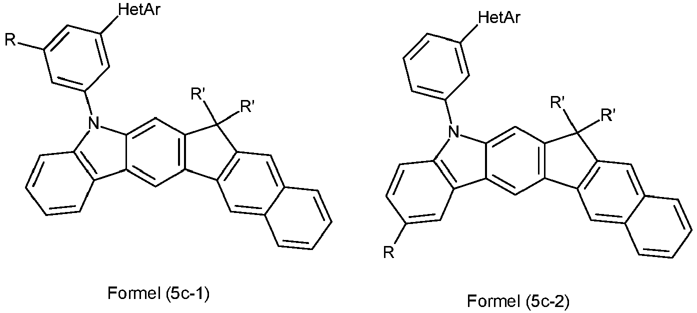 wobei HetAr, R und R' die oben genannten Bedeutungen aufweisen.

Wie oben beschrieben, ist HetAr eine Gruppe gemäß einer der Formeln (HetAr-1) und (HetAr-3) bis (HetAr-5).

Dabei sind in den Gruppen der Formeln (HetAr-1) und (HetAr-3) bis (HetAr-5) bevorzugt maximal zwei N-Atome direkt aneinander gebunden. Besonders bevorzugt sind keine N-Atome direkt aneinander gebunden.

In einer bevorzugten Ausführungsform der Erfindung weisen die Gruppen (HetAr-1) und (HetAr-3) bis (HetAr-5) zwei oder drei N-Atome auf. Dabei ist für Formel (HetAr-1) bevorzugt, wenn diese eine Pyrimidingruppe oder eine 1,3,5-Triazingruppe darstellt. Für die Formeln (HetAr-3) und (HetAr-4) ist es bevorzugt, wenn diese zwei N-Atome aufweisen. Besonders bevorzugt stellt die Formel (HetAr-4) eine Chinazolingruppe dar.

Bevorzugt sind die Gruppen der Formeln (HetAr-1) und (HetAr-3), besonders bevorzugt die Gruppe der Formel (HetAr-1).

Bevorzugte Ausführungsformen der Gruppe (HetAr-1) sind die Gruppen der Formeln (HetAr-1a) bis (HetAr-1d), bevorzugte Ausführungsformen der Gruppe (HetAr-3) sind die Gruppen der Formel (HetAr-3a), bevorzugte Ausführungsformen der Gruppe (HetAr-4) sind die Gruppen der Formel (HetAr-4a) und bevorzugte Ausführungsformen der Gruppe (HetAr-5) sind die Gruppen der Formel (HetAr-5a) und (HetAr-5b), 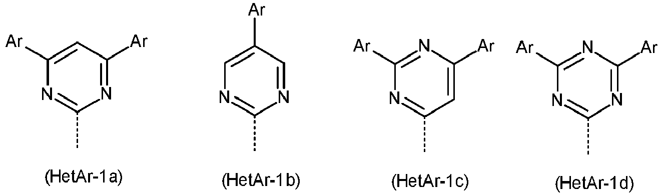 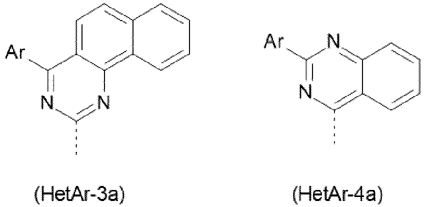 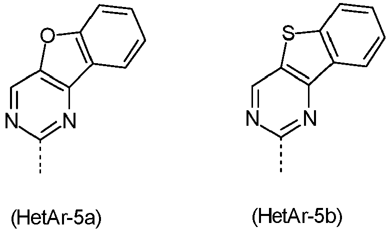 wobei Ar gleich oder verschieden bei jedem Auftreten für ein aromatisches oder heteroaromatisches Ringsystem mit 5 bis 40 aromatischen Ringatomen steht, welches mit einem oder mehreren Resten R¹ substituiert sein kann, und die weiteren Symbole die oben genannten Bedeutungen aufweisen.

Bevorzugte aromatische bzw. heteroaromatische Ringsysteme Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können.

Dabei sind die Gruppen Ar unabhängig voneinander besonders bevorzugt gewählt aus den Gruppen der folgenden Formeln Ar-1 bis Ar-75, 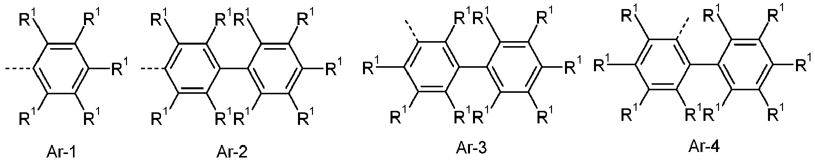 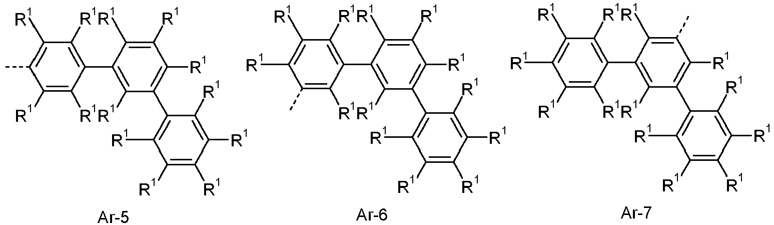 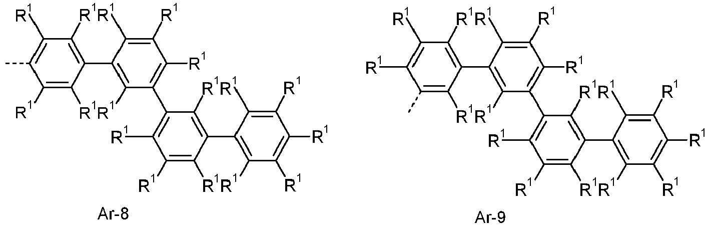 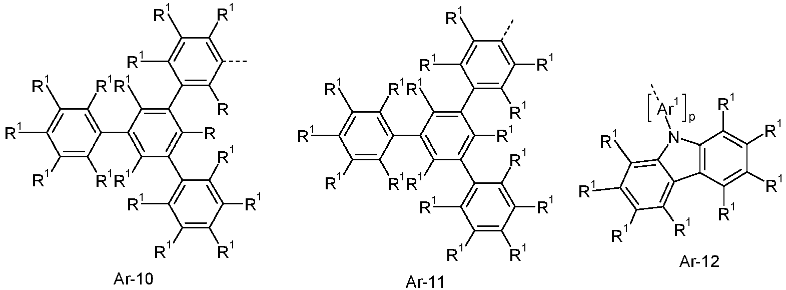 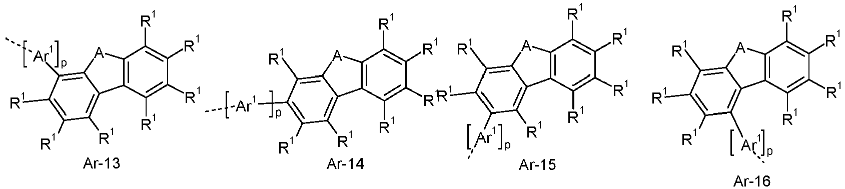 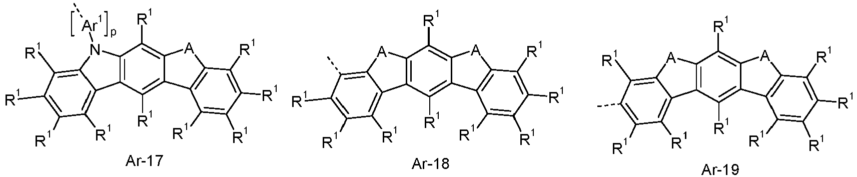 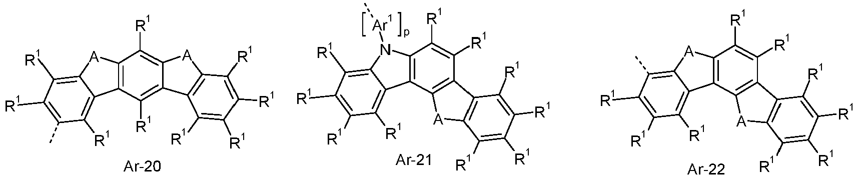 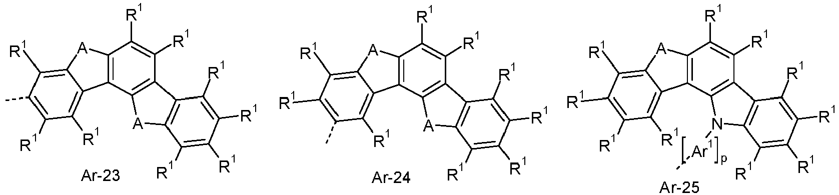 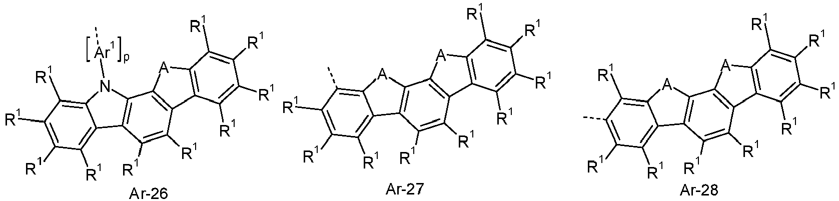 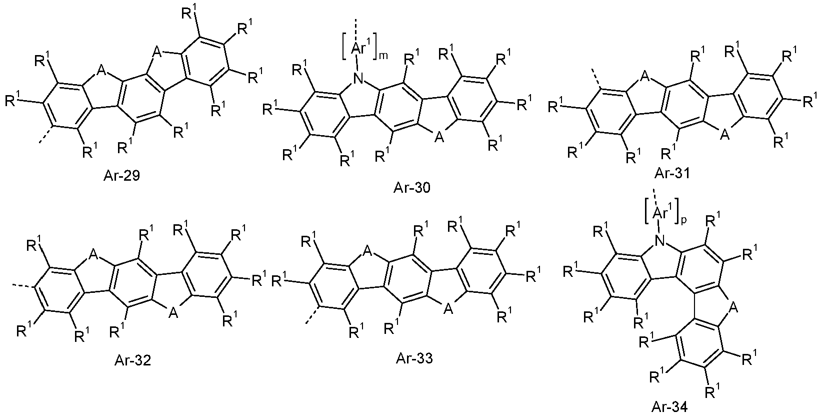 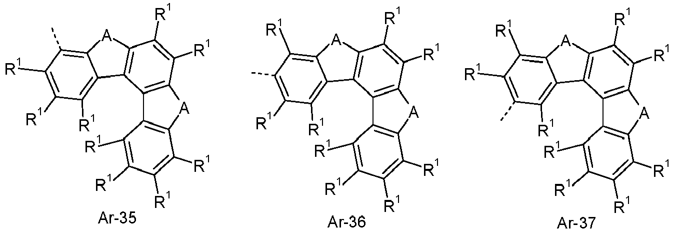 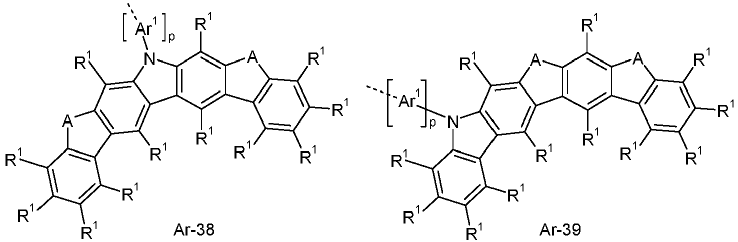 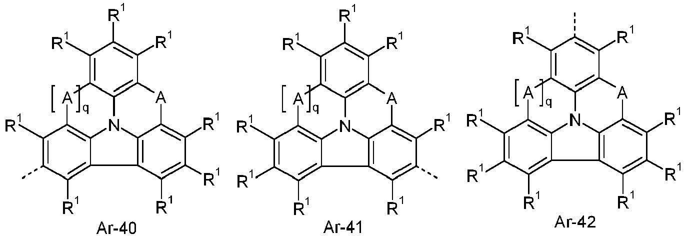 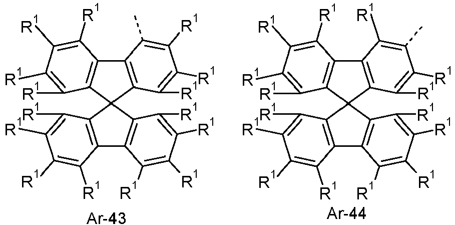 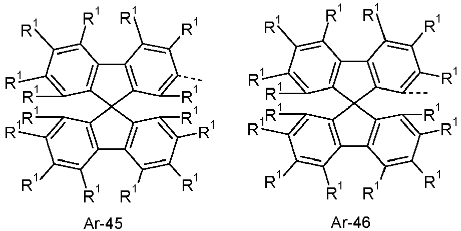 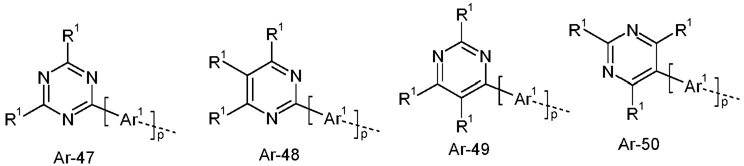 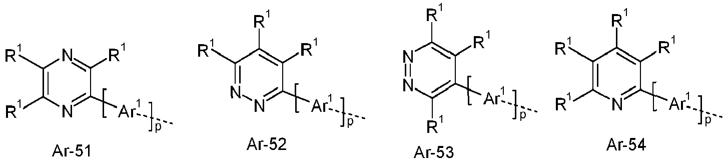 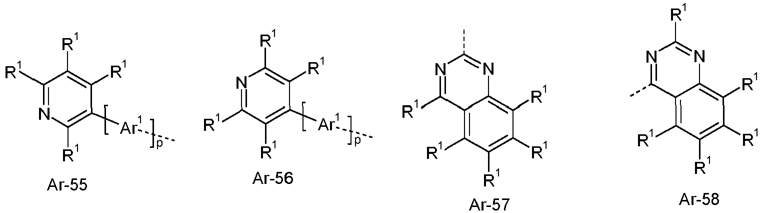 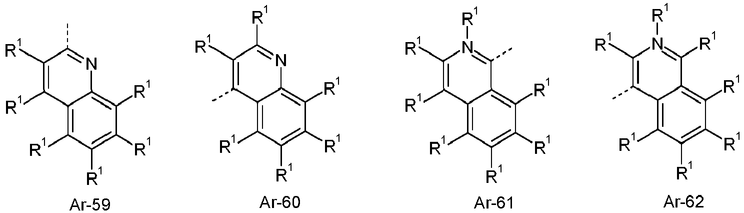 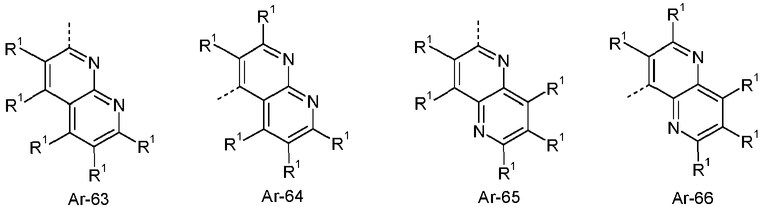 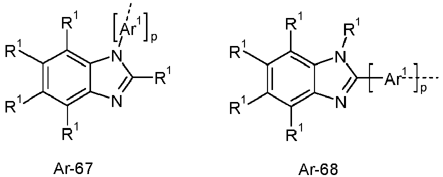 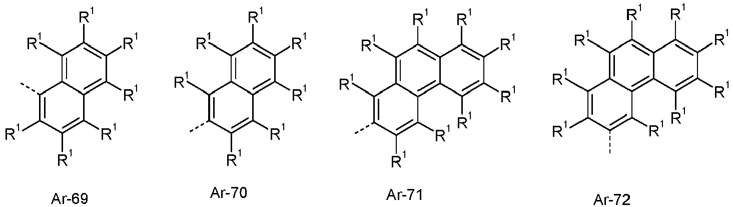 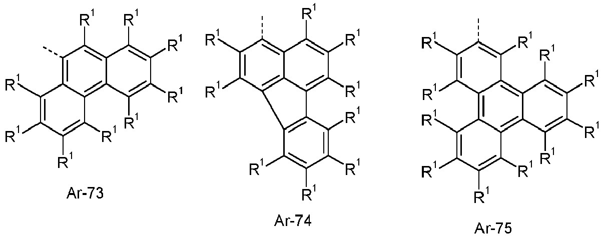 wobei R¹ die oben genannten Bedeutungen aufweist, die gestrichelte Bindung die Bindung an HetAr darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an HetAr gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Wenn die oben genannten Gruppen für Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Im Folgenden werden bevorzugte Substituenten R und R' beschrieben.

In einer bevorzugten Ausführungsform der Erfindung ist R gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist R gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

Bevorzugte aromatische bzw. heteroaromatische Ringsystem R bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können. Besonders bevorzugt sind die oben aufgeführten Strukturen Ar-1 bis Ar-75.

Weitere geeignete Gruppen R sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3-oder 4-Carbazol, 1-, 2-, 3- oder 4-Dibenzofuran, 1-, 2-, 3-oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer bevorzugten Ausführungsform der Erfindung ist R' bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R¹ substituiert sein kann, oder einem aromatischen oder heteroaromatischen Ringsystem mit 6 bis 24 aromatischen Ringatomen, das jeweils durch einen oder mehrere Reste R¹ substituiert sein kann; dabei können zwei Reste R' auch miteinander ein Ringsystem bilden. Besonders bevorzugt ist R' bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R¹ substituiert sein kann, bevorzugt aber unsubstituiert ist; dabei können zwei Reste R' miteinander ein Ringsystem bilden. Ganz besonders bevorzugt ist R' bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf. Beispiele für bevorzugte Verbindungen gemäß den oben aufgeführten Ausführungsformen sind die in der folgenden Tabelle aufgeführten Verbindungen.

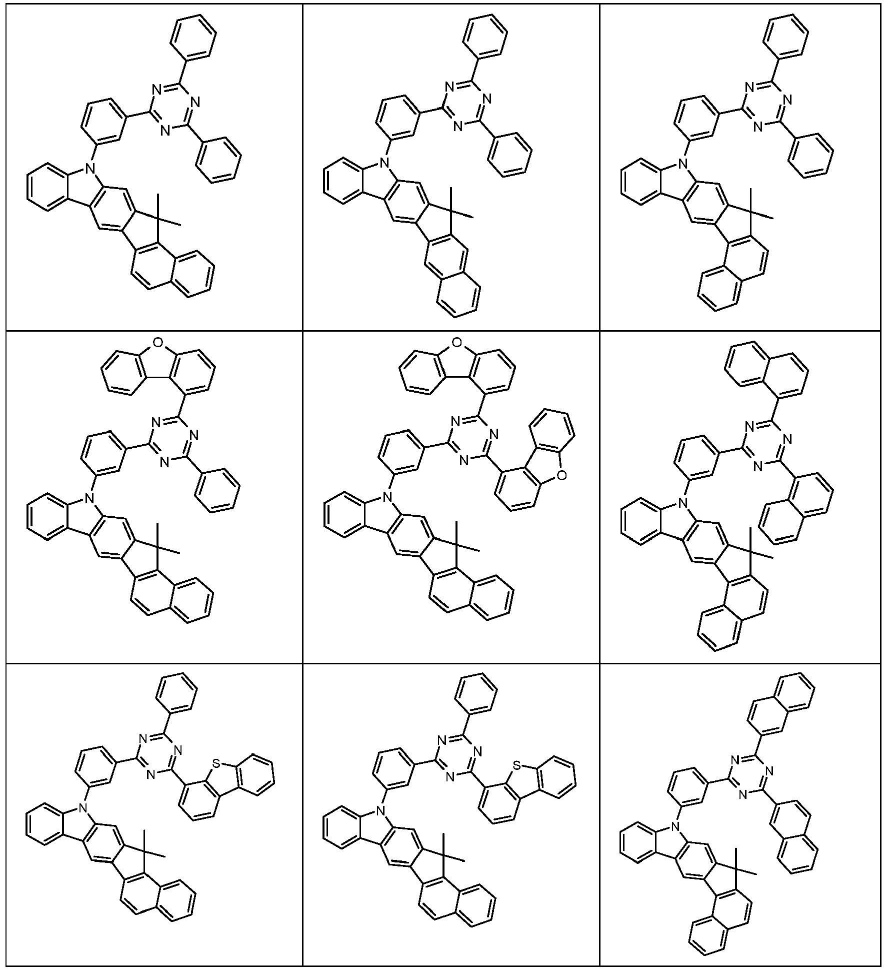

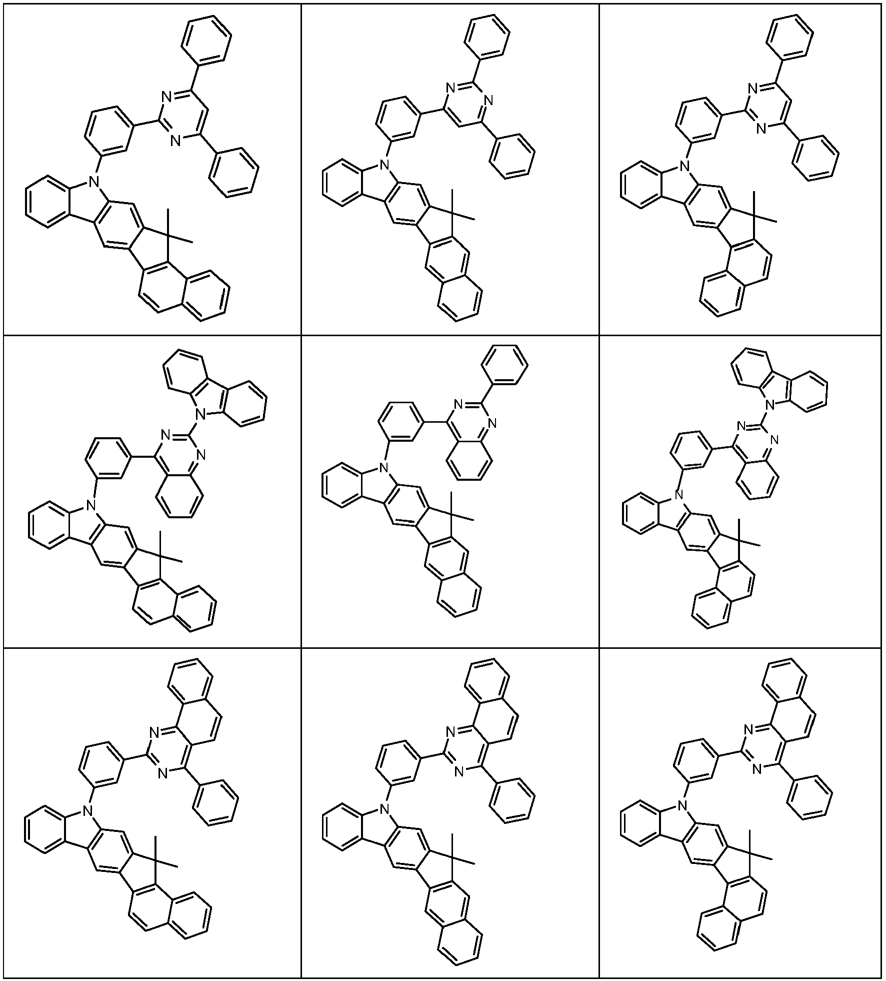

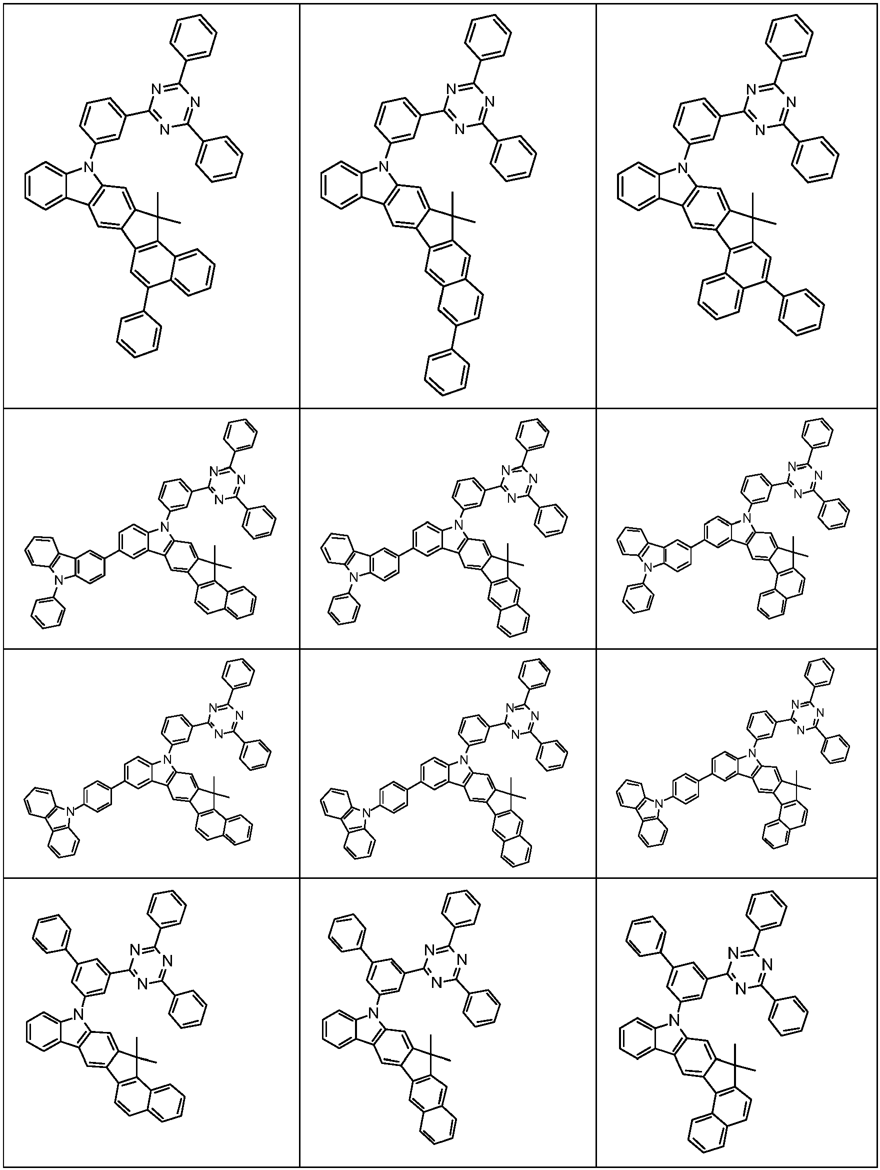

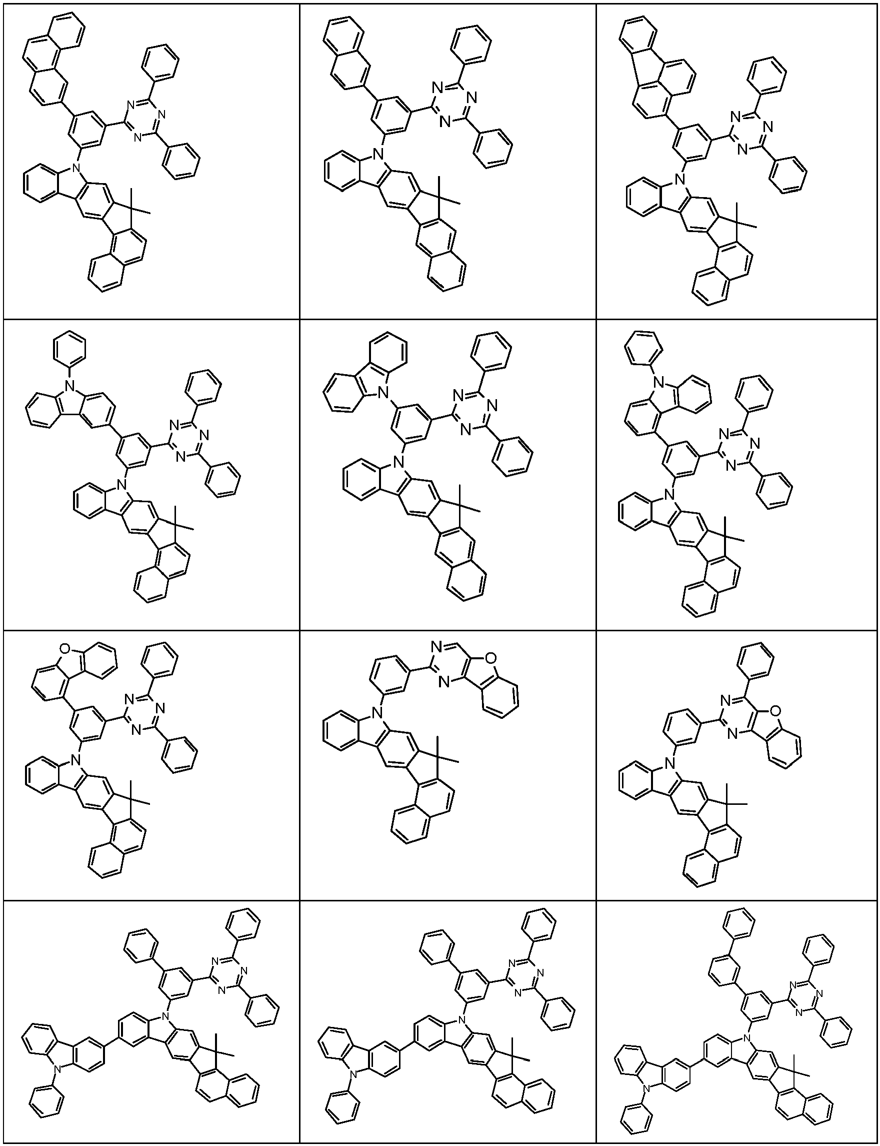

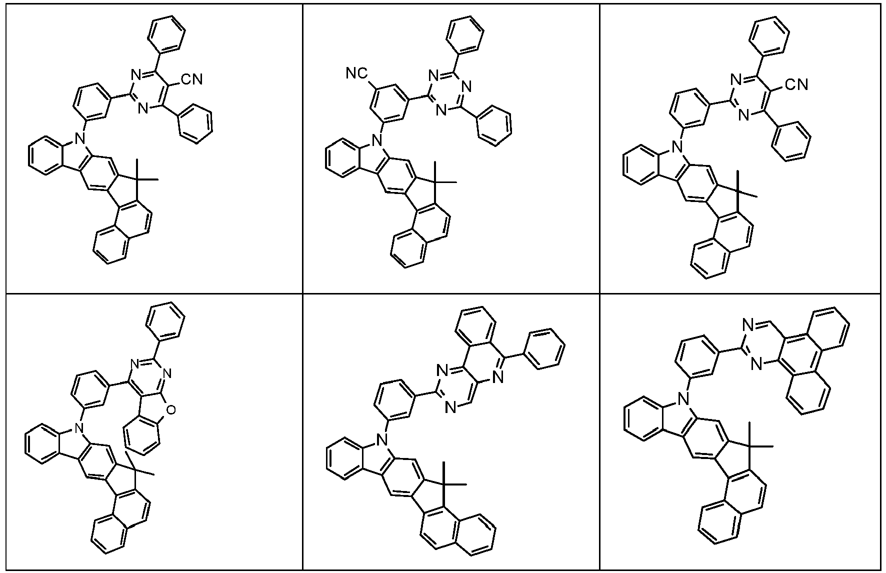

Die Grundstruktur der erfindungsgemäßen Verbindungen kann nach den in den nachfolgenden Schemata skizzierten Wegen dargestellt werden. Dabei sind die einzelnen Syntheseschritte, wie beispielsweise C-C-Kupplungsreaktionen gemäß Suzuki, C-N-Kupplungsreaktionen gemäß Hartwig-Buchwald oder Cyclisierungsreaktionen, dem Fachmann prinzipiell bekannt. Weitere Informationen zur Synthese der erfindungsgemäßen Verbindungen können den Synthesebeispielen entnommen werden. Die Synthese der Grundstruktur ist in Schema 1 dargestellt. Diese kann durch Kupplung eines Benzfluorens, welches mit einer reaktiven Abgangsgruppe, beispielsweise Brom, substituiert ist, mit einer gegebenenfalls substituierten 2-Nitrobenzolboronsäure, gefolgt von einer Ringschlussreaktion erfolgen. Alternativ kann die Kupplung mit der Aminogruppe eines gegebenenfalls substituierten 2-Aminochlorbenzol, gefolgt von einer Ringschlussreaktion erfolgen. In Schema 2 und 3 werden verschiedene Möglichkeiten der Einführung der Gruppe m-Phenylen-HetAr am Stickstoffatom des Grundgerüsts dargestellt, wobei HetAr für eine Gruppe gemäß einer der Formeln (HetAr-1) bzw. (HetAr-3) bis (HetAr-5) steht. Dabei kann eine mit einer geeigneten Abgangsgruppe, beispielweise Brom, substituierte Gruppe m-Phenylen-HetAr in einer nukleophilen aromatischen Substitution oder eine Palladium-katalysierten Kupplungsreaktion eingeführt werden, wie in Schema 2 dargestellt. Alternativ kann zunächst in einer nukleophilen aromatischen Substitution die m-Phenylengruppe, welche noch eine geeignete Abgangsgruppe, beispielsweise Brom, trägt, am Grundgerüst eingeführt werden und in einer weiteren Kupplungsreaktion, gegebenenfalls nach Umwandlung in ein Boronsäurederivat, die Gruppe (HetAr-1) bzw. (HetAr-3) bis (HetAr-5) eingeführt werden, wie in Schema 3 dargestellt an einer heteroaromatischen Gruppe, die nicht Gegenstand der Erfindung ist.

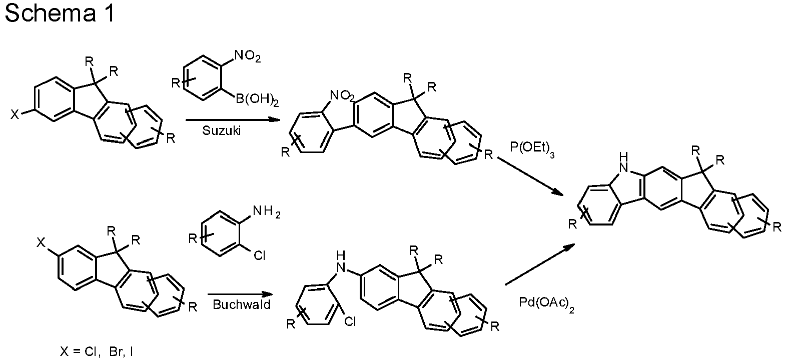

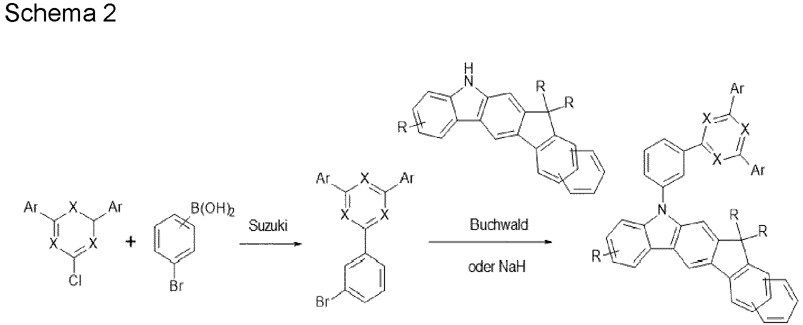

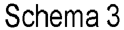

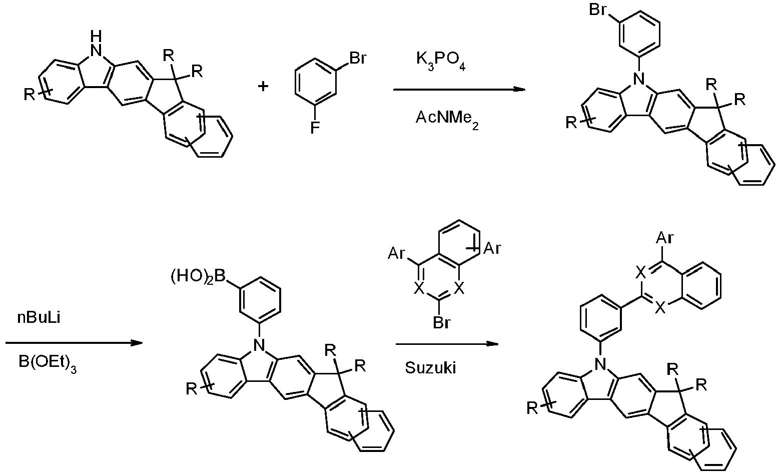

Ein weiterer Gegenstand der vorliegenden Erfindung ist daher ein Verfahren zur Herstellung einer erfindungsgemäßen Verbindung, wobei zunächst das Grundgerüst synthetisiert wird, welches die Gruppe meta-Phenylen-HetAr noch nicht enthält, wobei HetAr für eine Gruppe gemäß einer der Formeln (HetAr-1) bzw. (HetAr-3) bis (HetAr-5 steht) und wobei die Gruppe meta-Phenylen-HetAr mittels einer nukleophilen aromatischen Substitutionsreaktion oder einer Kupplungsreaktion eingeführt wird.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Die weitere Verbindung kann auch polymer sein.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial für rot, orange oder gelb phosphoreszierende Emitter, insbesondere für rot phosphoreszierende Emitter, in einer emittierenden Schicht oder als Elektronentransport- bzw. Lochblockiermaterial in einer Elektronentransport- bzw. Lochblockierschicht eingesetzt.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung dabei als einziges Matrixmaterial ("single host") für den phosphoreszierenden Emitter eingesetzt.

Eine weitere Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Ebenso kann ein weiterer phosphoreszierender Emitter, welcher kürzerwellig als der eigentliche Emitter emittiert, als Co-Host in der Mischung vorhanden sein. Besonders gute Ergebnisse werden erzielt, wenn als Emitter ein rot phoshoreszierender Emitter eingesetzt wird und als Co-Host in Kombination mit der erfindungsgemäßen Verbindung ein gelb phosphoreszierender Emitter verwendet wird.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-MatrixMaterial Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Besonders bevorzugte Co-Host-Materialien, welche in Kombination mit den erfindungsgemäßen Verbindungen eingesetzt werden können, sind Biscarbazol-Derivate gemäß einer der Formeln (6) oder (7), 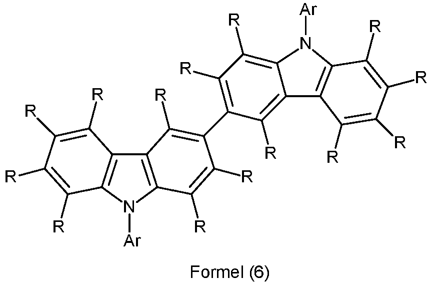 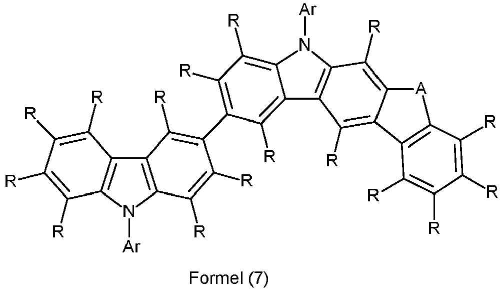 wobei Ar und A die oben genannten Bedeutungen aufweisen und R die oben genannten Bedeutungen aufweist, jedoch Reste R hier auch miteinander ein aromatisches oder heteroaromatisches Ringsystem bilden können. In einer bevorzugten Ausführungsform der Erfindung steht A für C(R')₂.

Bevorzugte Ausführungsformen der Verbindungen der Formeln (6) bzw. (7) sind die Verbindungen der folgenden Formeln (6a) bzw. (7a), 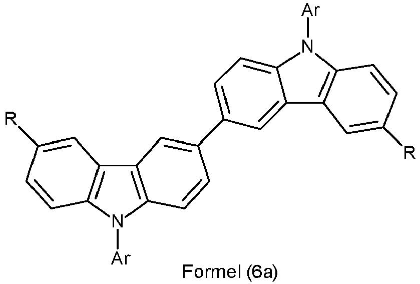 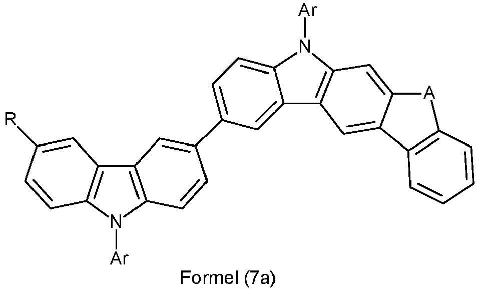 wobei die verwendeten Symbole die oben genannten Bedeutungen aufweisen.

Beispiele für geeignete Verbindungen gemäß Formel (6) oder (7) sind die nachfolgend abgebildeten Verbindungen.

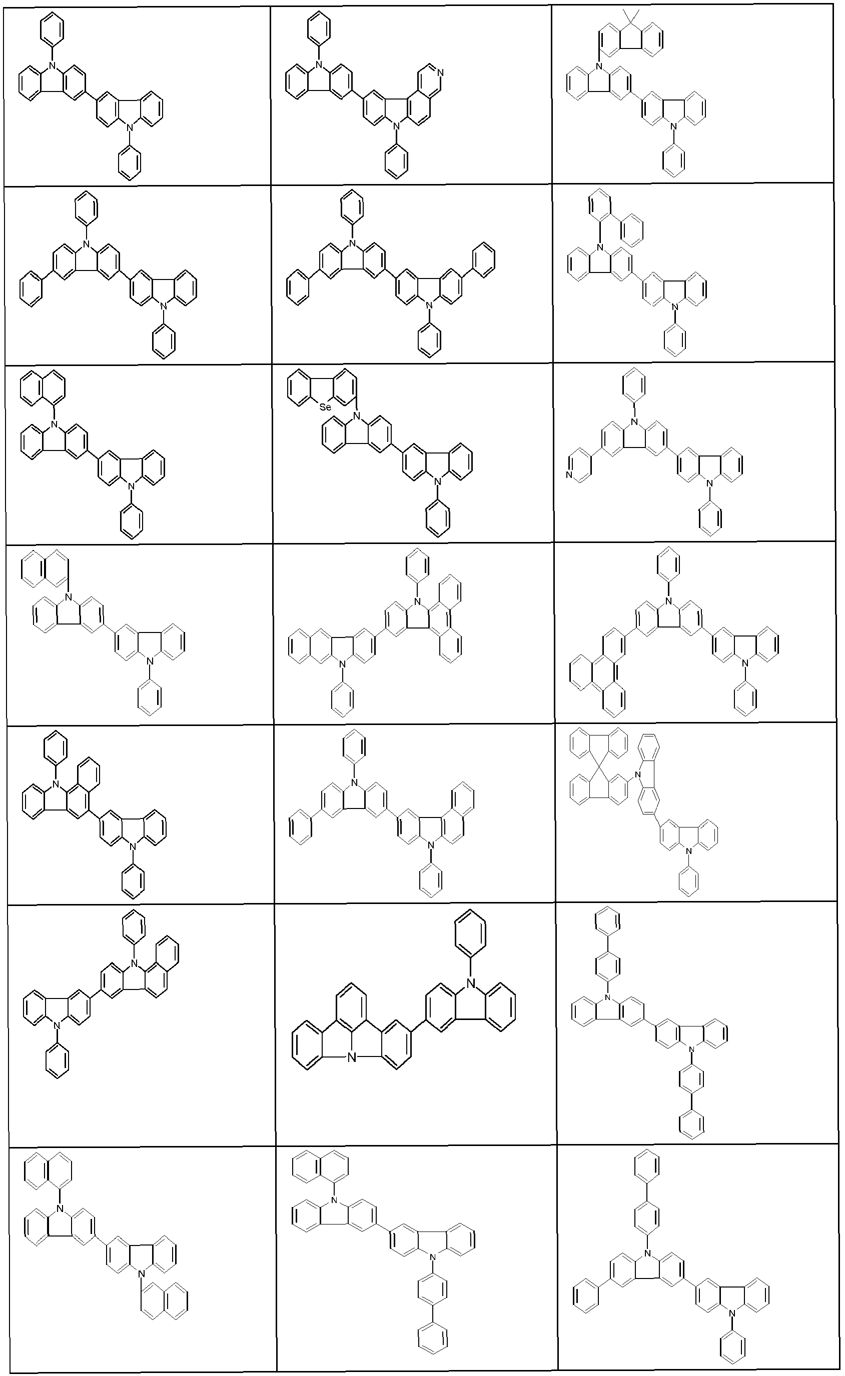

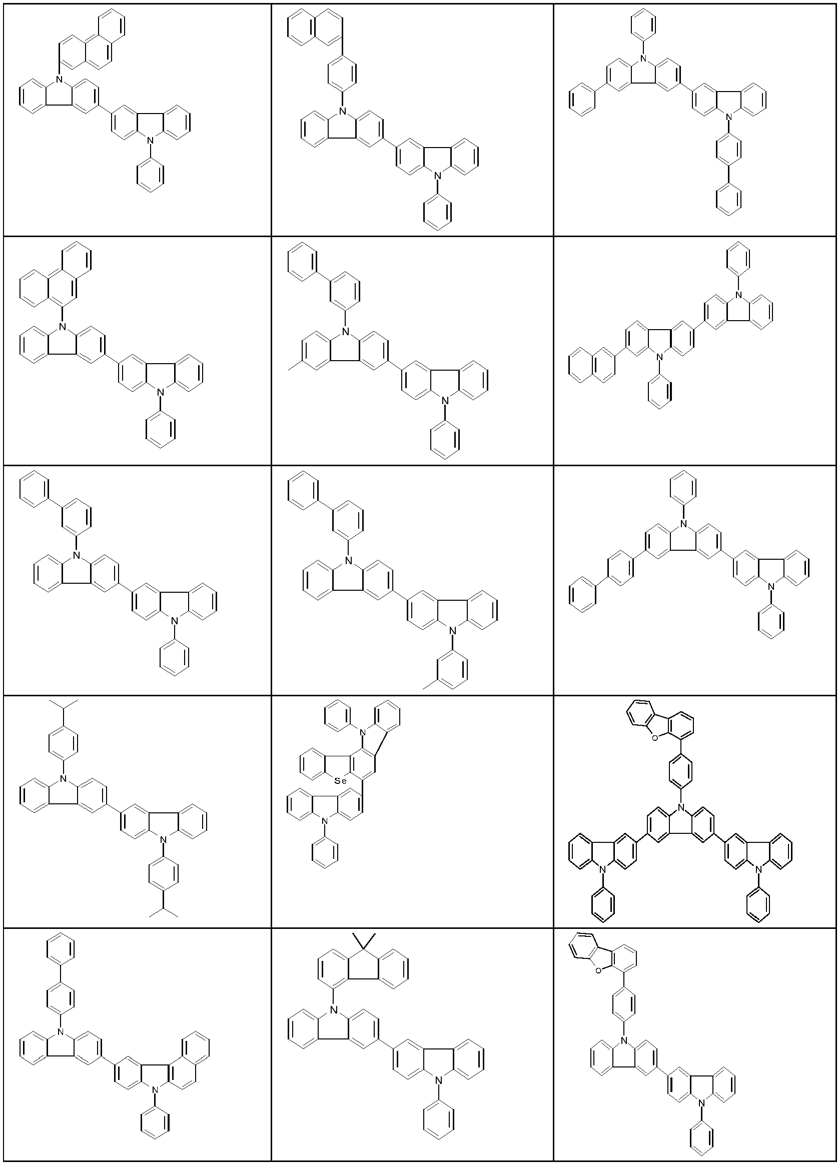

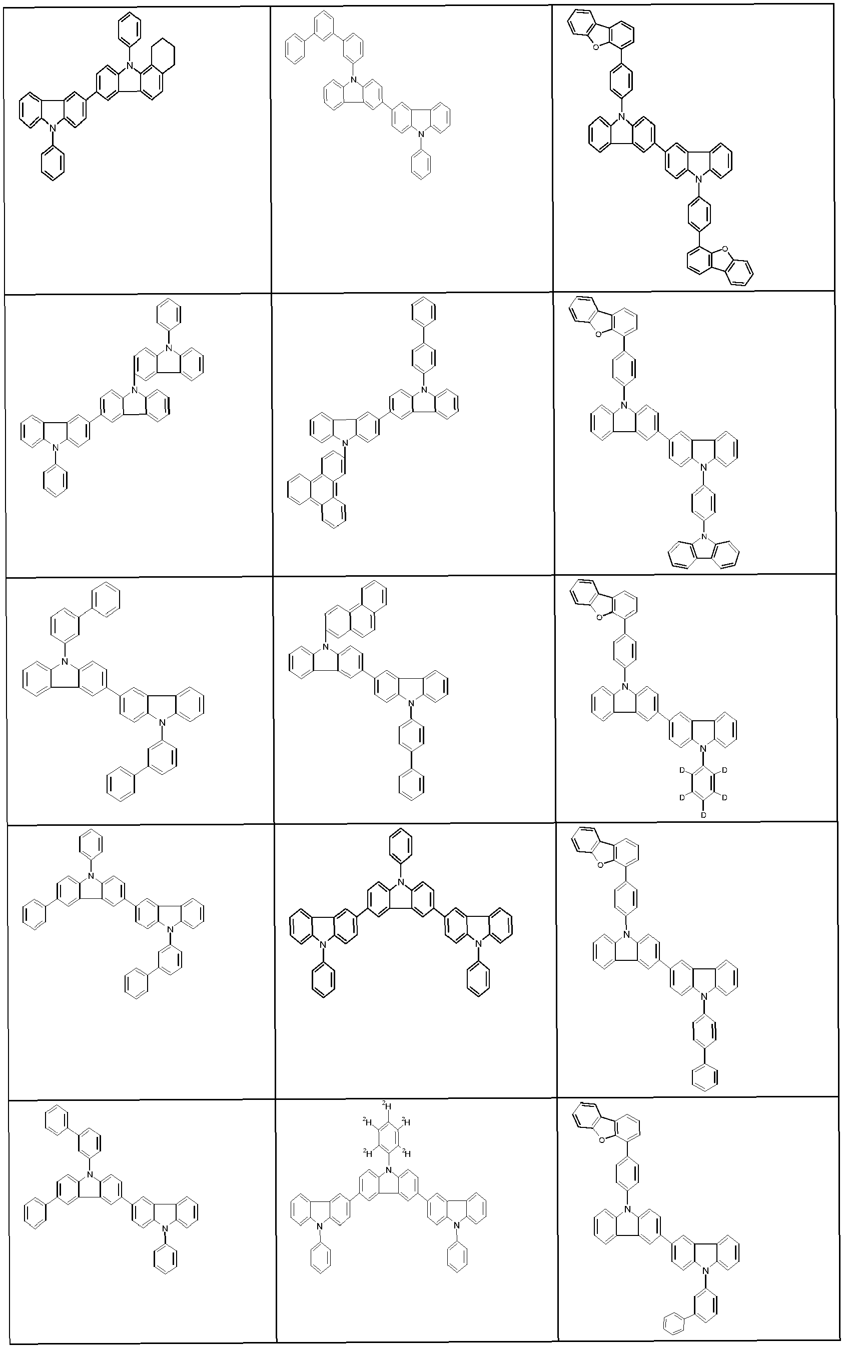

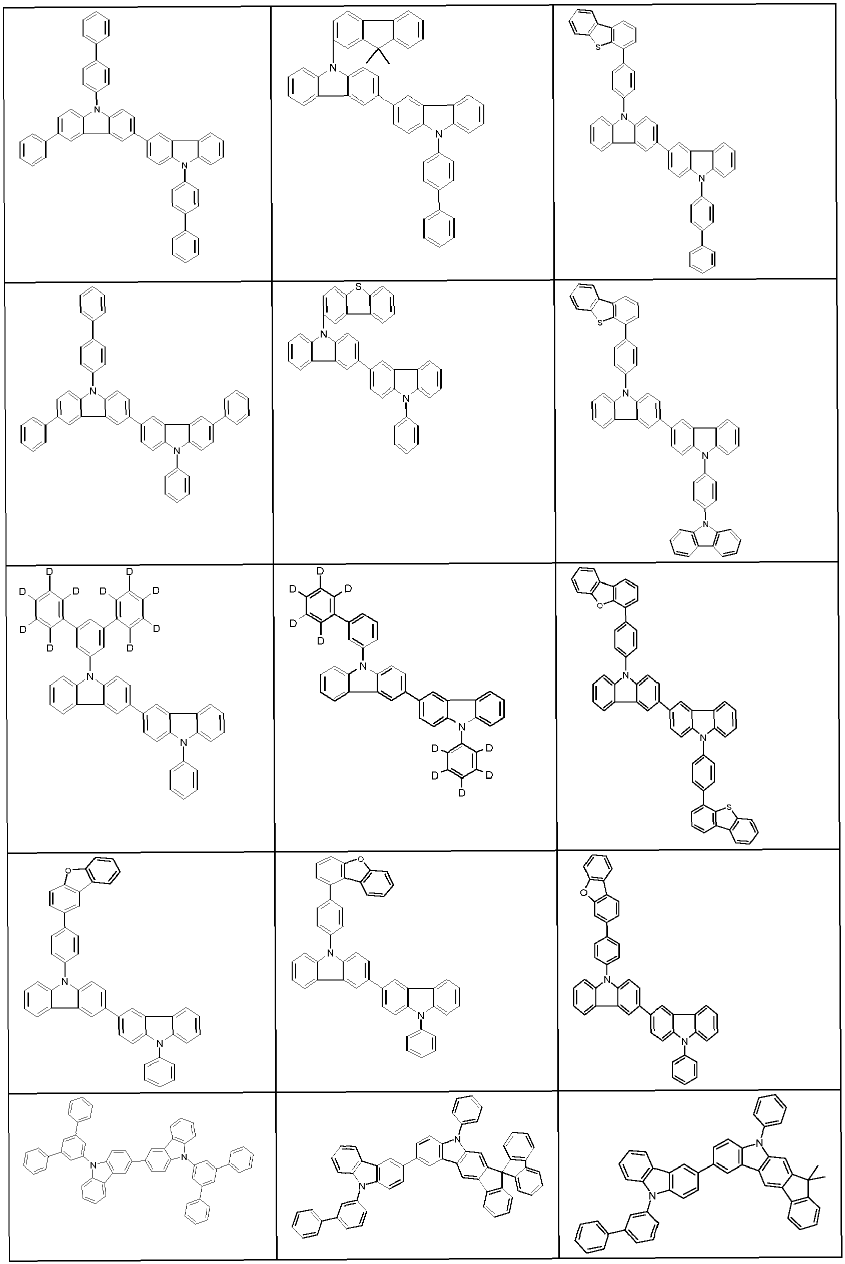

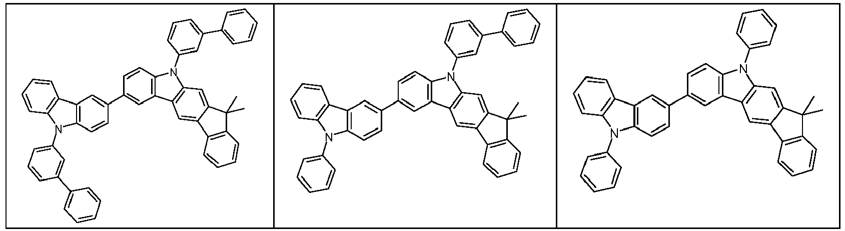

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und WO 2018/011186 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind in der folgenden Tabelle aufgeführt.

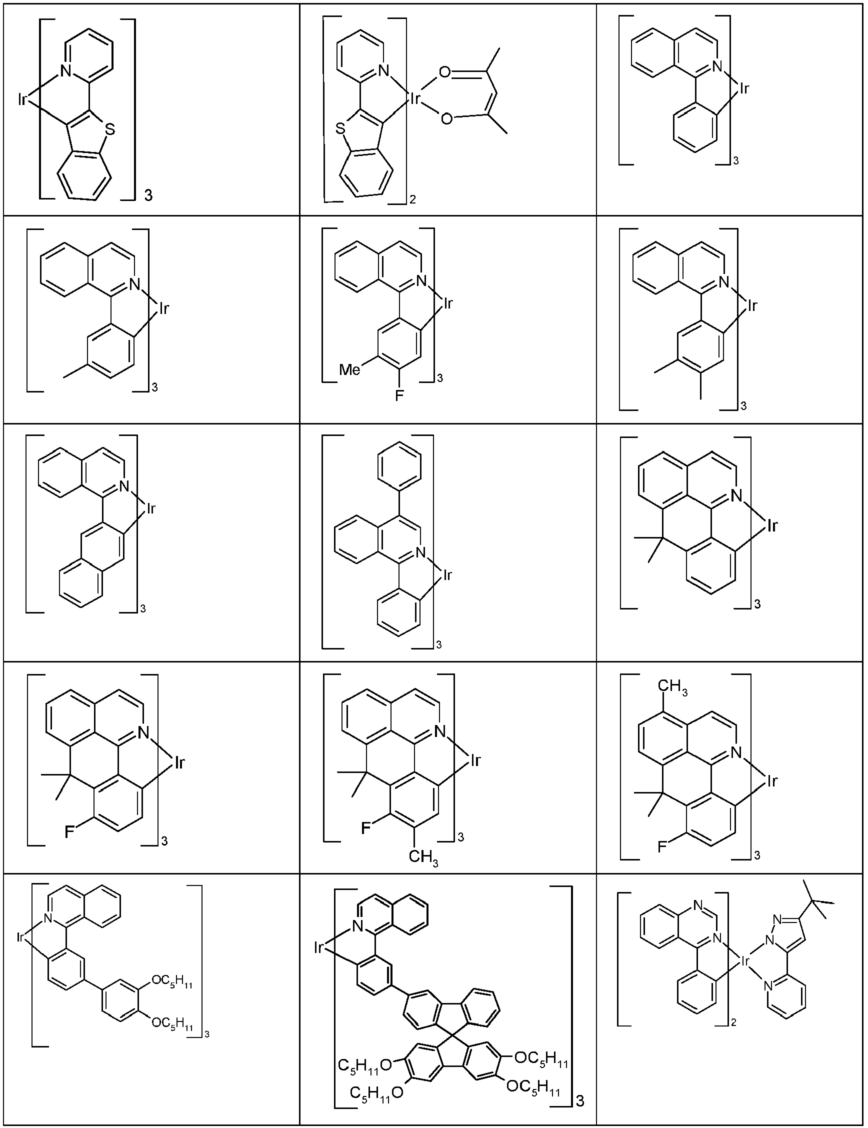

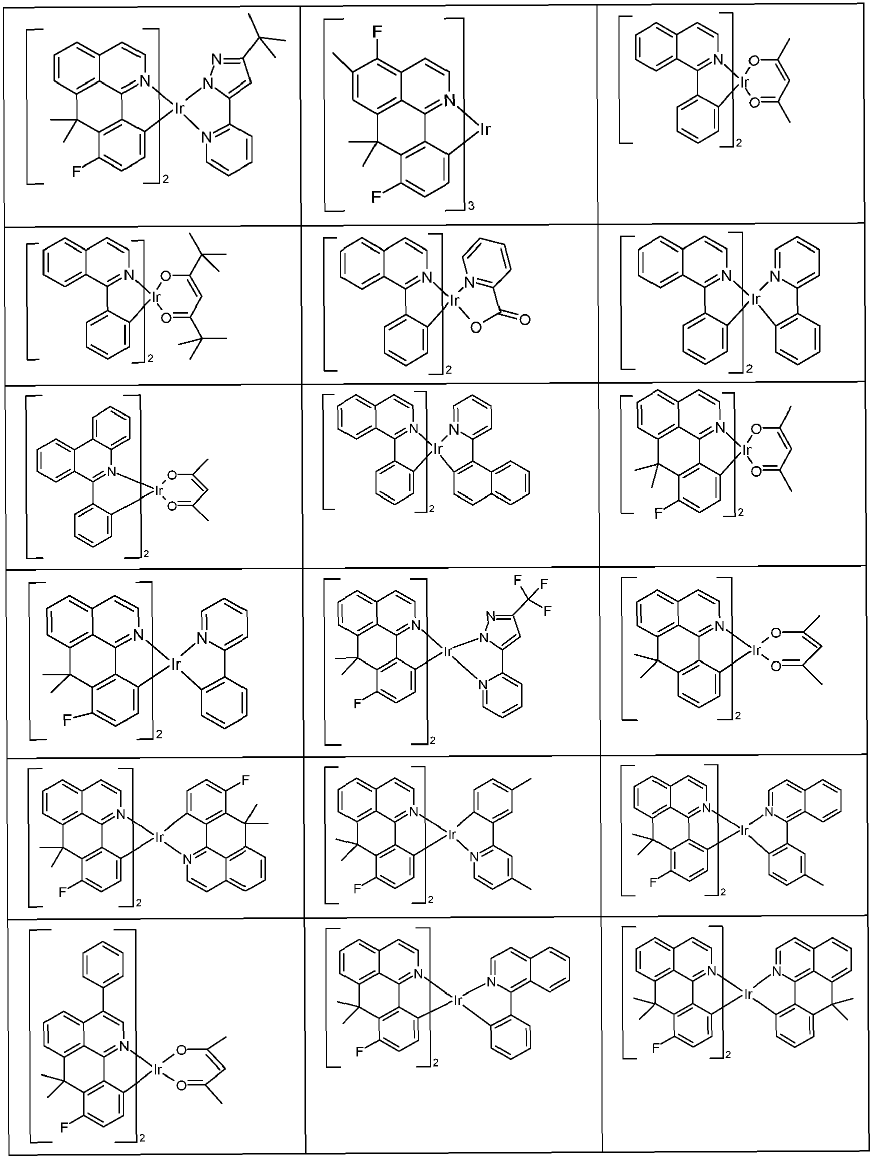

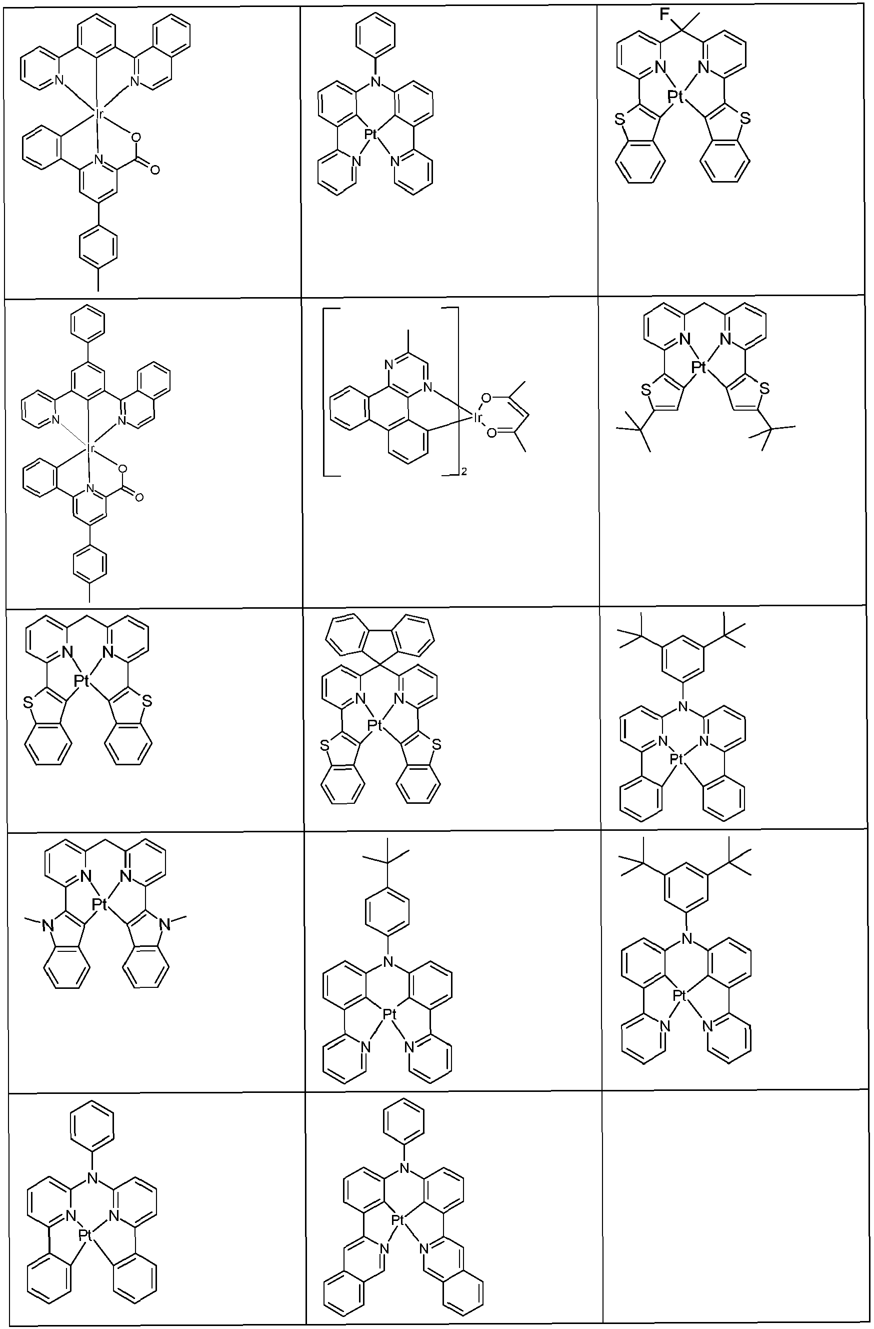

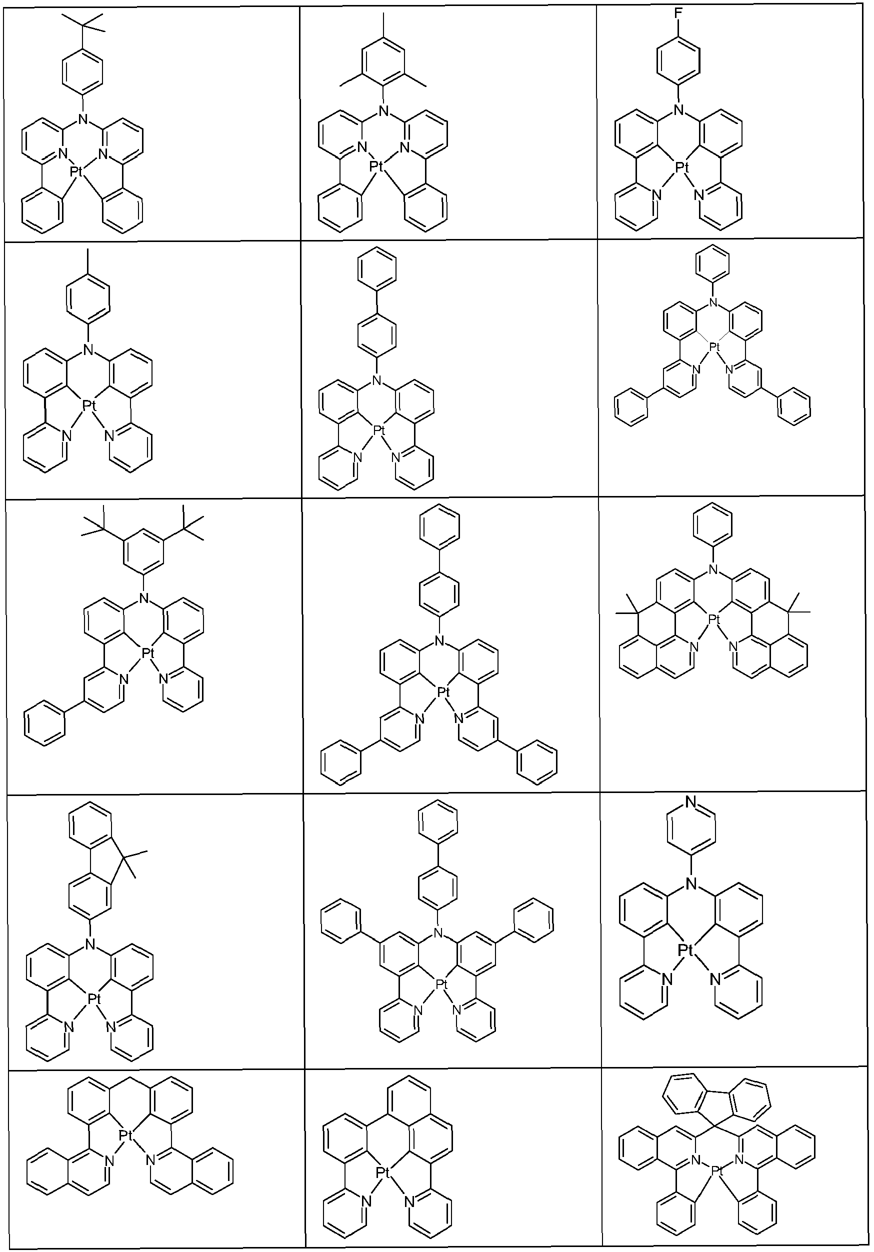

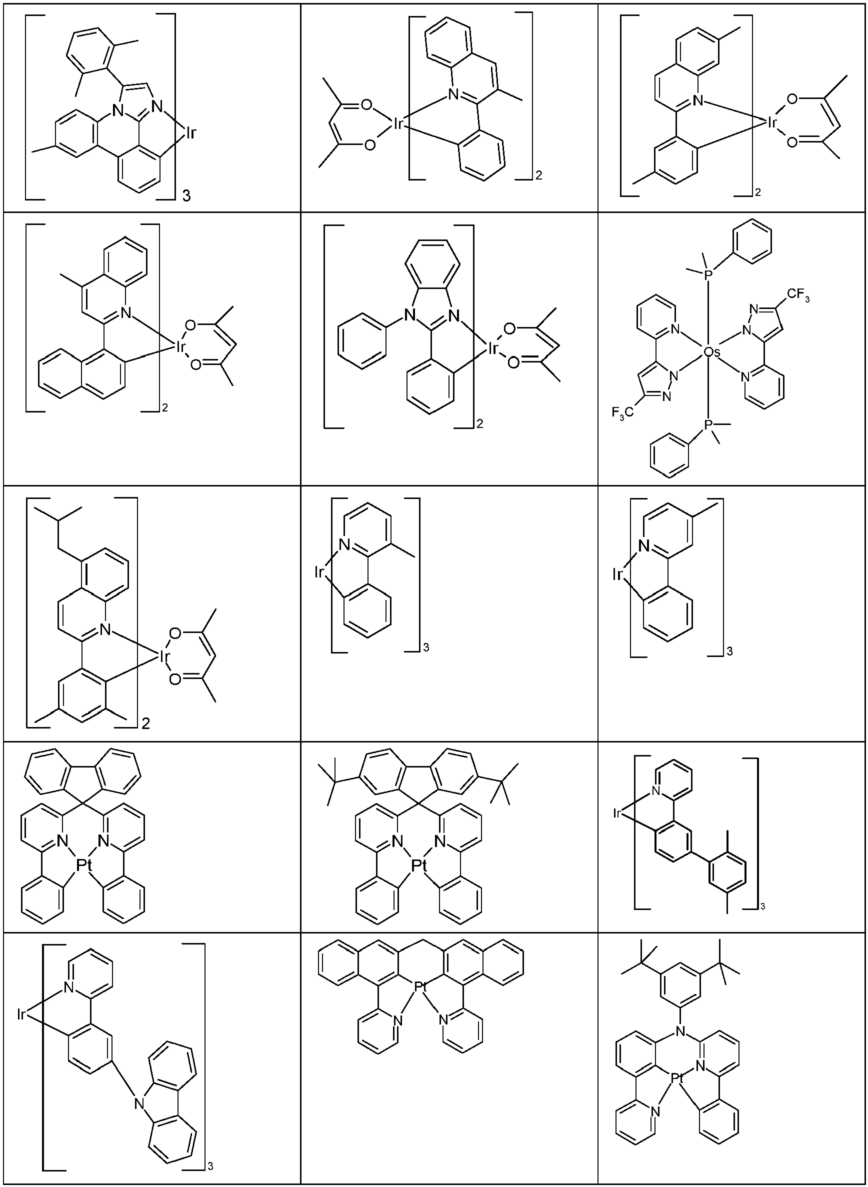

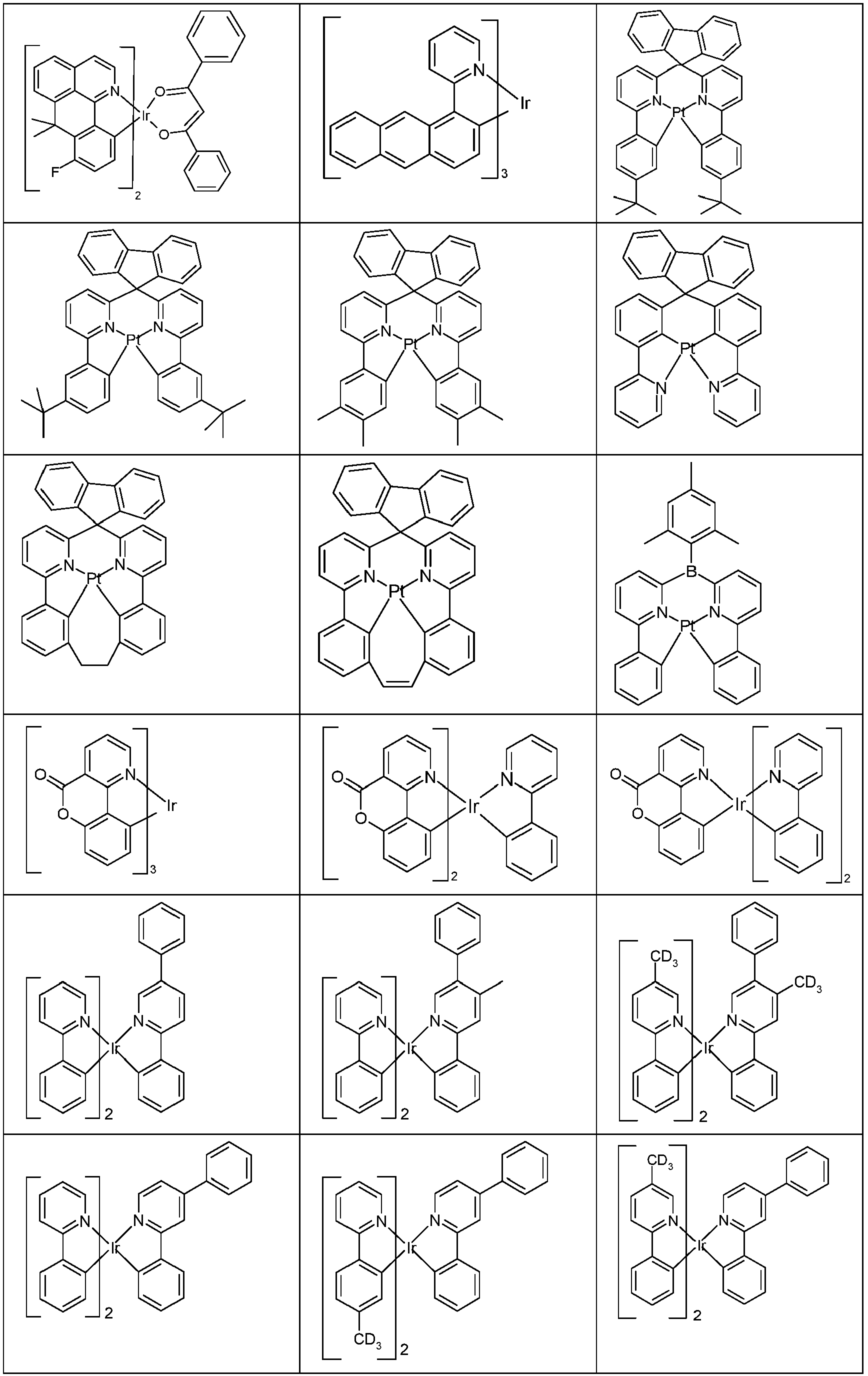

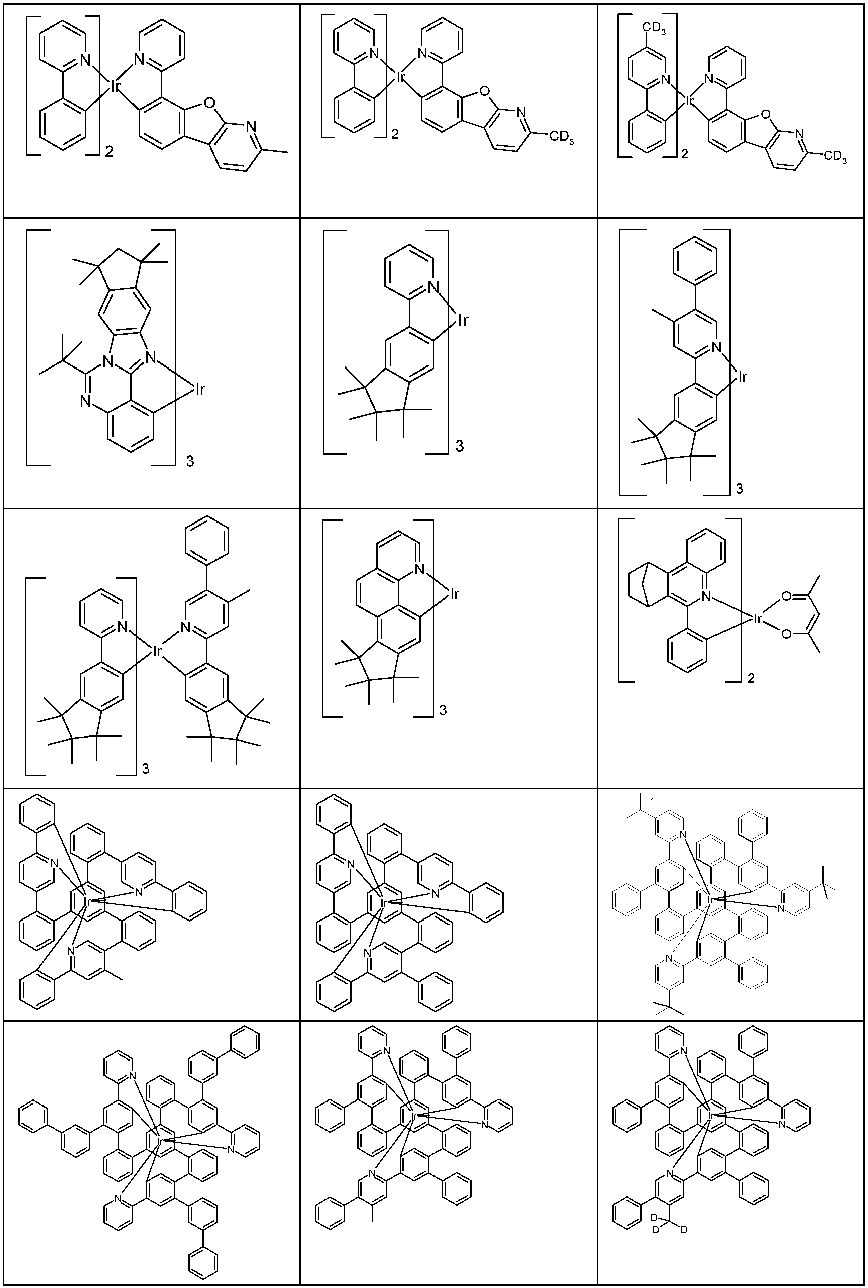

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer aus. Dies gilt vor allem gegenüber ähnlichen Verbindungen, welche statt des Benzindenocarbazol-Grundgerüsts ein Indenocarbazol-Grundgerüst aufweisen. Dabei bleiben die weiteren elektronischen Eigenschaften der OLED, wie Effizienz oder Betriebsspannung, mindestens gleich gut.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) (2-Chlorphenyl)-(11,11-dimethyl-11H-benzo[a]fluoren-9-yl)-amin

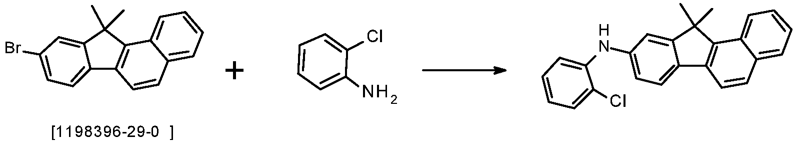

47 g (145 mmol) 9-Brom-11,11-dimethyl-11H-benzo[a]fluoren, 16.8 g (159 mmol) 2-Chloranilin, 41.9 g (436.2 mmol) Natrium-tert-butylat, 1.06 g (1.45 mmol) Pd(dppf)Cl₂ werden in 500 ML Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 33 g (89 mmol); 70% d.Th..

Analog können folgende Verbindungen hergestellt werden:

| | **Edukt 1** | **Edukt 2** | **Produkt** | **Ausbeute** |
|---|---|---|---|---|
| 1a | 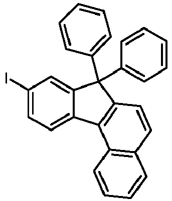 | 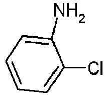 | 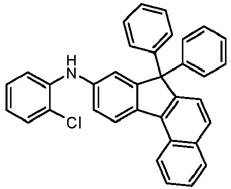 | 79% |
| | [1804905-31-4 ] | | | |
| 2a | 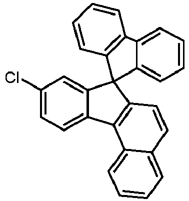 | 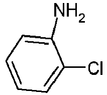 | 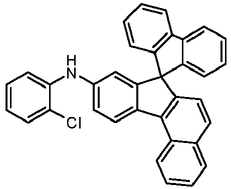 | 77% |
| | [1800333-59-8 ] | | | |
| 3a | 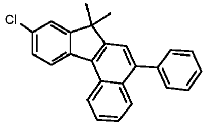 | 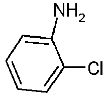 | 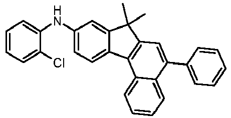 | 78% |
| | [1263204-40-5 ] | | | |
| 4a | 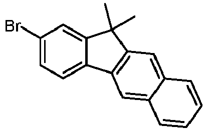 | 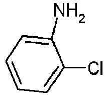 | 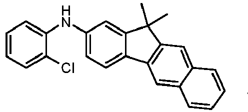 | 79% |
| | [1198396-39-2 ] | | | |
| 5a | 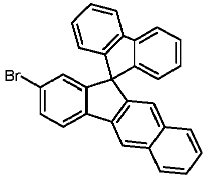 | 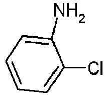 | 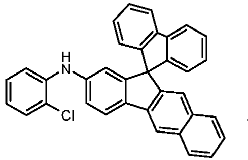 | 74% |
| 6a | 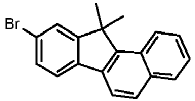 | 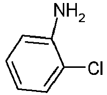 | 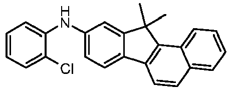 | 81% |
| | [1198396-29-0 ] | | | |
| 7a | 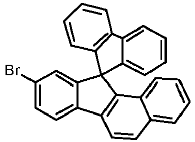 | 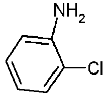 | 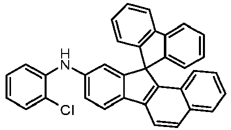 | 78% |
| | [1198396-35-8 ] | | | |
| 8a | 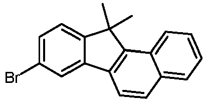 | 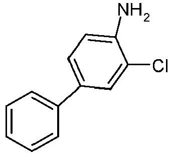 | 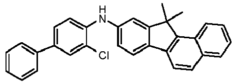 | 77% |
| | [1674335-13-7 ] | | | |

### b) Cyclisierung

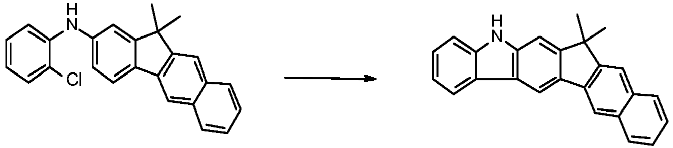

48 g (129 mmol) (2-Chlorphenyl)-(11,11-dimethyl-11H-benzo[a]fluoren-9-yl)-amin, 53 g (389 mmol) Kaliumcarbonat, 4.5 g (12 mmol) Tricyclohexylphosphintetrafluoroborat, 1.38 g (6 mmol) Palladium(II)acetat und 3.3 g (32 mmol) Pivalinsäure werden in 500 mL Dimethylacetamid suspendiert und 6 h unter Rückfluss gerührt. Nach Erkalten wird die Reaktionsmischung mit 300 mL Wasser und 400 mL CH₂Cl₂ versetzt. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 34 g (102 mmol); 78% d.Th..

Analog können folgende Verbindungen hergestellt werden:

| | **Edukt** | **Produkt** | **Ausbeute** |
|---|---|---|---|
| 1b | 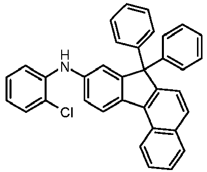 | 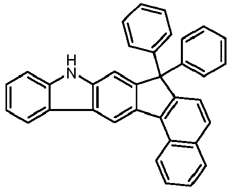 | 79% |
| 2b | 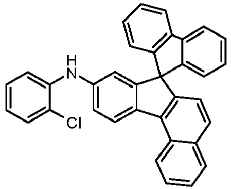 | 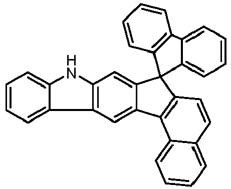 | 77% |
| 3b | 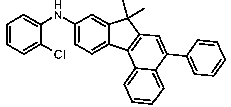 | 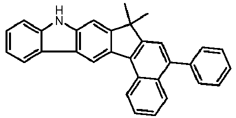 | 78% |
| 4b | 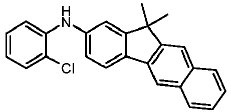 | 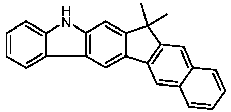 | 75% |
| 5b | 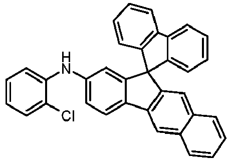 | 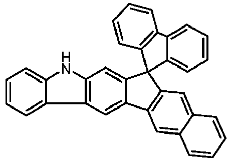 | 78% |
| 6b | 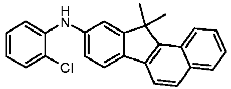 | 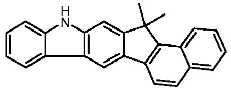 | 73% |
| 7b | 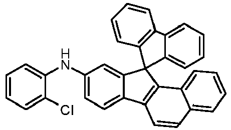 | 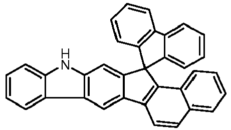 | 71% |
| 8b | 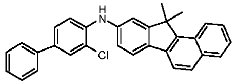 | 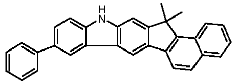 | 76% |

### c) 11,11-Dimethyl-3-(2-nitro-phenyl)-11H-benzo[b]fluoren

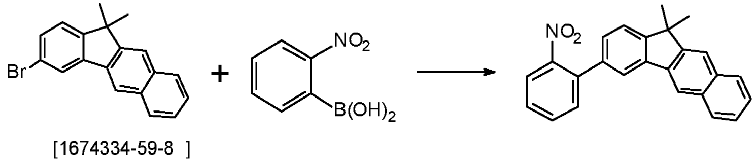

Eine gut gerührte, entgaste Suspension aus 59 g (183.8 mmol) 2-Nitrobenzolboronsäure, 54 g (184 mmol) 3-Brom-11,11-dimethyl-11H-benzo[b]-fluoren und 66.5 g (212.7 mmol) Kaliumcarbonat in einem Gemisch aus 250 mL Wasser und 250 mL THF wird mit 1.7 g (1.49 mmol) Pd(PPh₃)₄ versetzt und 17 h unter Rückfluss erhitzt. Nach Erkalten wird die organische Phase abgetrennt, dreimal mit je 200 mL Wasser und einmal mit 200 mL gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet. Ausbeute: 53 g (146 mmol); 80% d. Th..

Analog können folgende Verbindungen hergestellt werden:

| | **Edukt 1** | **Edukt 2** | **Produkt** | **Ausbeute** |
|---|---|---|---|---|
| 1c | 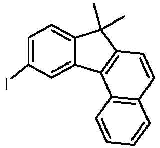 | 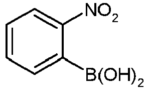 | 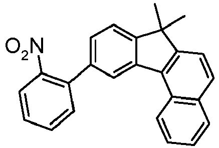 | 74% |
| | [1927921-26-3 ] | | | |
| 2c | 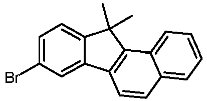 | 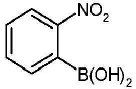 | 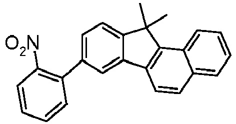 | 77% |
| | [1674335-13-7 ] | | | |

### d) Carbazolsynthese

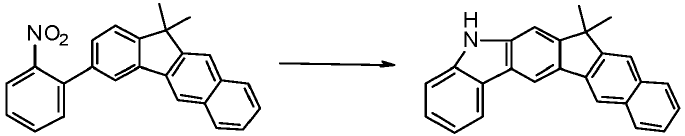

Eine Mischung aus 87 g (240 mmol) 11,11-Dimethyl-3-(2-nitro-phenyl)-11H-benzo[b]fluoren und 290.3 mL (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert. Ausbeute: 58 g (176 mmol); 74 % d. Th..

Analog können folgende Verbindungen hergestellt werden:

| | **Edukt** | **Produkt** | **Ausbeute** |
|---|---|---|---|
| 1d | 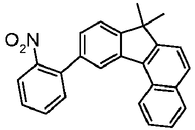 | 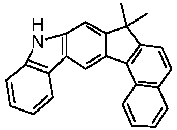 | 79% |
| 2d | 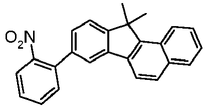 | 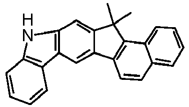 | 76% |

### e) Nukleophile Substitution

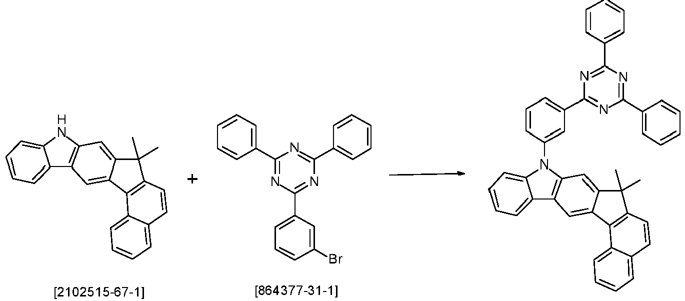

4.2 g (106 mmol) NaH, 60 %ig in Mineralöl, werden in 300 mL Dimethylformamid (DMF) unter Schutzatmosphäre gelöst. 35 g (106 mmol) 7,9-Dihydro-7,7-dimethyl-benz[6,7]indeno[2,1-*b*]carbazol werden in 250 mL DMF gelöst und zu der Reaktionsmischung zugetropft. Nach 1 h bei Raumtemperatur wird eine Lösung von 31.4 g (122 mmol) 2-(3-Bromphenyl)-4,6-diphenyl-[1,3,5]triazin in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol/n-Heptan umkristallisiert und abschließend im Hochvakuum sublimiert. Die Reinheit beträgt 99.9%. Die Ausbeute beträgt 34 g (53 mmol); 66 % d. Th..

Analog können folgende Verbindungen hergestellt werden:

| | **Edukt 1** | **Edukt 2** | **Produkt** | **Ausbeute** |
|---|---|---|---|---|
| 1e | 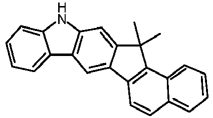 | 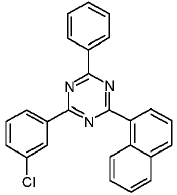 | 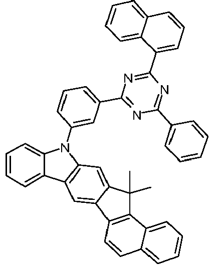 | 61% |
| | [213765-59-7] | [2120350-09-4] | | |
| 2e | 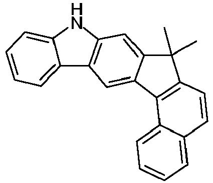 | 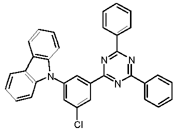 | 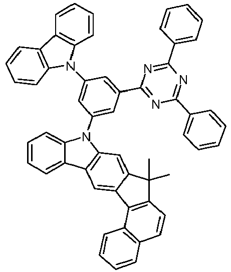 | 62% |
| | [2102515-67-1] | [2097261-60-2] | | |
| 3e | 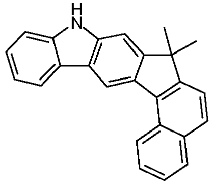 | 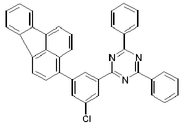 | 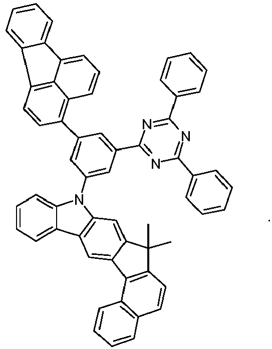 | 58% |
| | [2102515-67-1] | [1040010-77-0] | | |
| 4e | 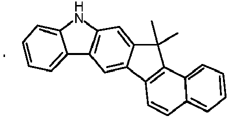 | 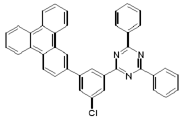 | 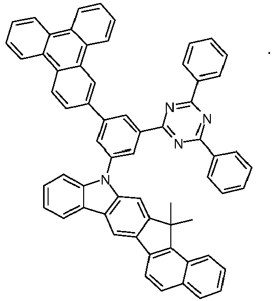 | 65% |
| | [213765-59-7] | [1824702-22-8] | | |
| 5e | 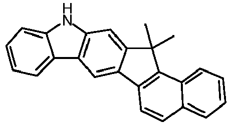 | 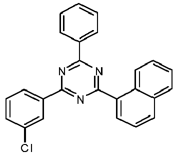 | 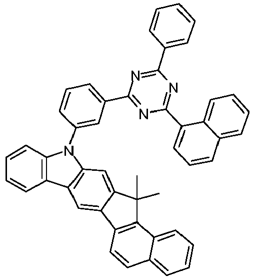 | 63% |
| | [213765-59-7] | [1891018-83-9] | | |
| 6e | 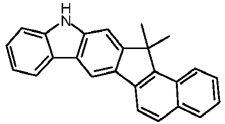 | 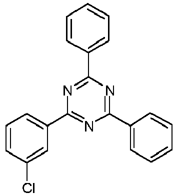 | 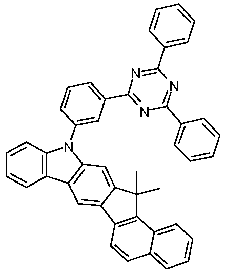 | 63% |
| | [213765-59-7] | [307929-32-4] | | |
| 7e | 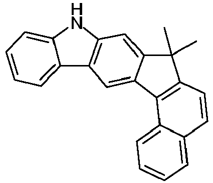 | 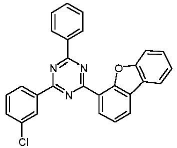 | 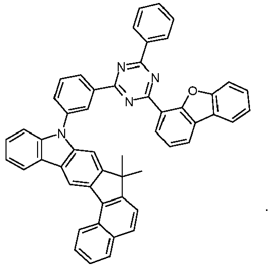 | 72% |
| | [2102515-67-1] | [2098802-13-0] | | |
| 8e | 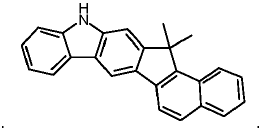 | 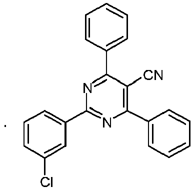 | 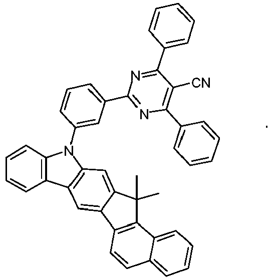 | 62% |
| | [213765-59-7] | [2129160-65-0] | | |
| 9e | 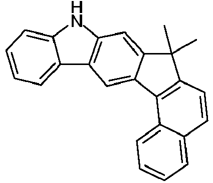 | 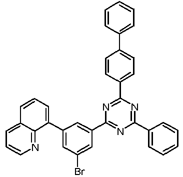 | 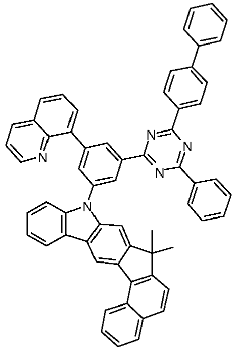 | 62% |
| | [2102515-67-1] | [2136352-17-3] | | |
| 10e | 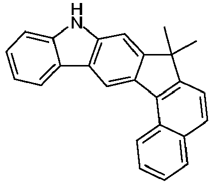 | 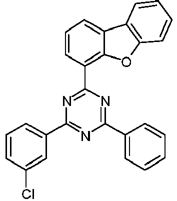 | 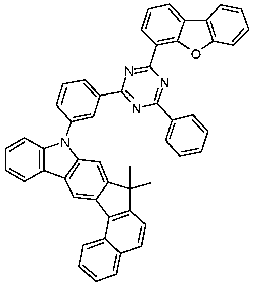 | 61% |
| | [2102515-67-1] | [2137919-54-9] | | |
| 11e | 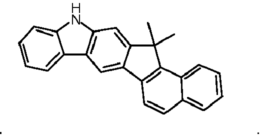 | 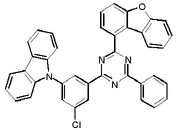 | 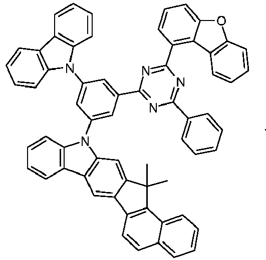 | 67% |
| | [213765-59-7] | [2097261-60-2] | | |
| 12e | 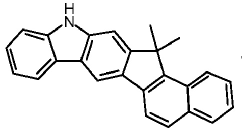 | 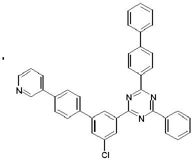 | 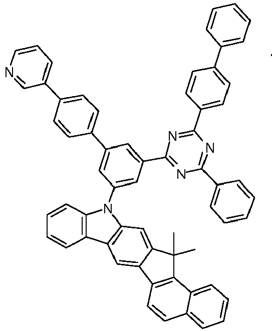 | 60% |
| | [213765-59-7] | [213652-15-1] | | |
| 13e | 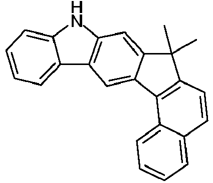 | 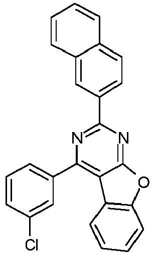 | 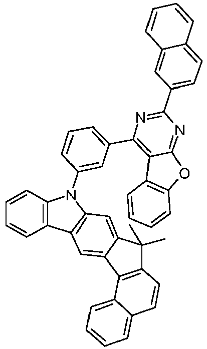 | 64% |
| | [2102515-67-1] | [2084128-82-3] | | |
| 14e | 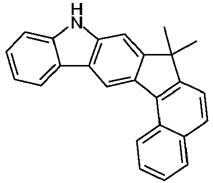 | 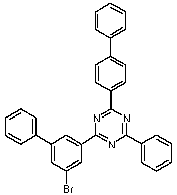 | 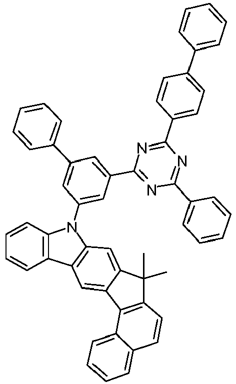 | 68% |
| | [2102515-67-1] | [2081938-92-1] | | |
| 15e | 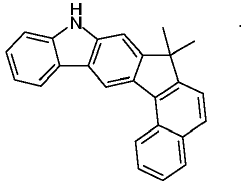 | 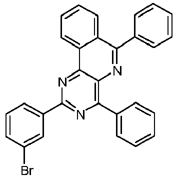 | 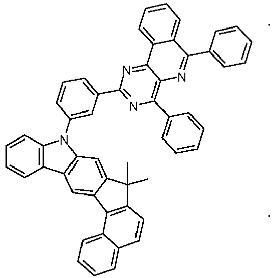 | 62% |
| | [2102515-67-1] | [1927896-61-4] | | |
| 20e | 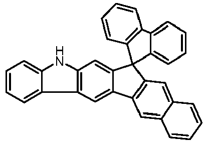 | 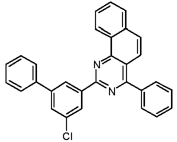 | 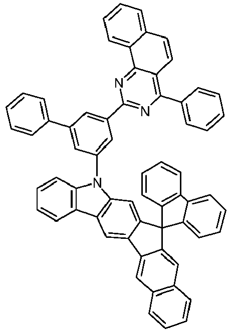 | 67% |
| | | [1831900-45-8] | | |
| 21e | 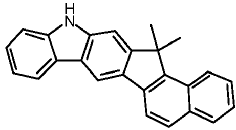 | 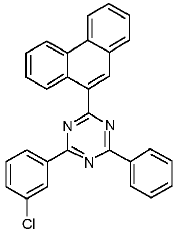 | 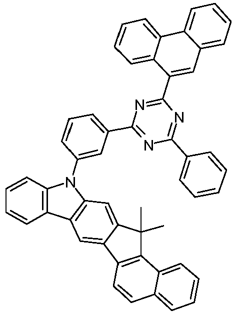 | 66% |
| | [213765-59-7] | [1927896-61-4] | | |
| 22e | 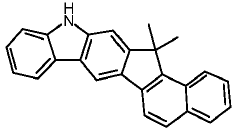 | 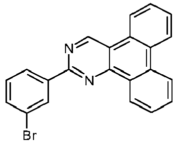 | 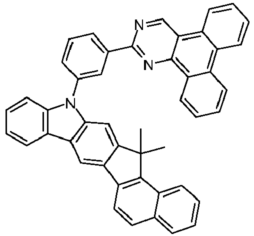 | 62% |
| | [213765-59-7] | [1957206-82-4] | | |
| 23e | 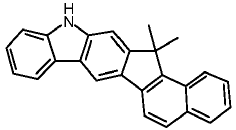 | 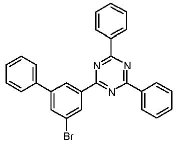 | 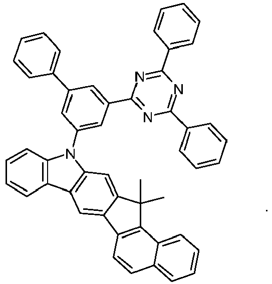 | 61% |
| | [213765-59-7] | [1394937-38-2 ] | | |
| 24e | 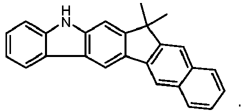 | 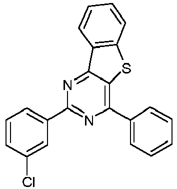 | 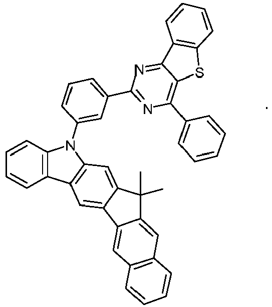 | 77% |
| | | [1801325-72-3] | | |
| 25e | 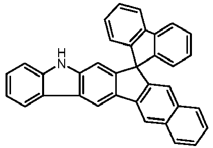 | 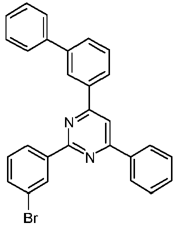 | 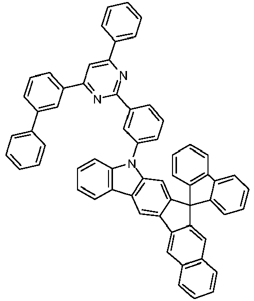 | 78% |
| | | [2036122-81-1] | | |

### f) Nukleophile Substitution

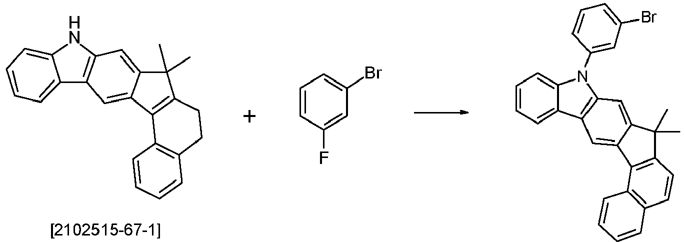

175.0 g (525 mmol) 7,9-Dihydro-7,7-dimethyl-benz[6,7]indeno[2,1-b] carbazol, 183.0 g (1.0 mmol) 1-Brom-3-fluorbenzol [1073-06-9] und 334.7 g (1.58 mol) Kaliumphosphat werden in 2 L Dimethylacetamid vorgelegt und 14 h unter Rückfluss erhitzt. Nach Abkühlen auf Raumtemperatur wird das Lösungsmittel am Rotationsverdampfer so weit wie möglich entfernt. Es verbleibt ein dunkelbraunes Öl. Nach kräftigem Anreiben der Kolbenwand mit einem Glasstab kann das Produkt durch langsames Einrühren von etwa 750 mL Ethanol ausgefällt werden. Der gebildete Feststoff wird abgesaugt, viermal mit je 250 mL Ethanol gewaschen, im Vakuum getrocknet und abschließend bei einem Druck von etwa 10⁻⁵ mbar bei 250 °C fraktioniert sublimiert. Ausbeute: 187 g (364 mmol); 73 % d. Th..

### g) Boronsäure-Synthese und anschließende Suzuki-Reaktion

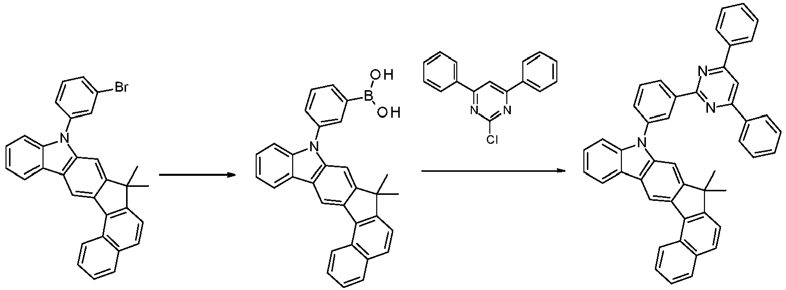

### Schritt 1:

92 g (190 mmol) des Produktes aus Beispiel f werden in 450 mL THF gelöst und auf -78 °C abgekühlt. Unter Rühren werden 100 mL n-Butyllithium (200 mmol, 2 M in Cyclohexan) so zugetropft, dass die Innentemperatur -65 °C nicht übersteigt. Nach 2 h werden 32.4 mL Trimethylborat (286 mmol) so zugetropft, dass die Innentemperatur -65 °C nicht übersteigt. Nach 2 h wird die Kühlung entfernt und das Gemisch weitere 16 h bei Raumtemperatur gerührt.

### Schritt 2:

53.3 g (200 mmol) 2-Chlor-4,6-diphenyl-pyrimidin und 40.4 g (381 mmol) Natriumcarbonat werden in einer Mischung aus 550 mL Toluol, 250 mL Wasser und 250 mL Ethanol vorgelegt. Die Suspension wird 30 Minuten mit Argon durchspült, dann werden 8.0 g (30 mmol) Triphenylphosphin und 1.7 g (8 mmol) Palladium(II)acetat zugegeben. Unter kräftigem Rühren wird die in Schritt 1 hergestellte Lösung zügig zugetropft und das Gemisch für 15 h unter Rückfluss erhitzt. Nach Abkühlen auf Raumtemperatur wird der gebildete Feststoff abgesaugt, im Vakuum getrocknet und anschließend zweimal mit je etwa 500 mL Toluol über Aluminiumoxid (basisch, Aktivitätsstufe 1) heißextrahiert. Der gebildete Feststoff wird mit etwa 350 mL Heptan ausgekocht. Der Rückstand wird mit Toluol heiß extrahiert, aus Toluol/n-Heptan umkristallisiert und abschließend im Hochvakuum sublimiert. Die Reinheit beträgt 99.9 %. Ausbeute: 60 g (94 mmol); 55 % d. Th..

### h) Bromierung

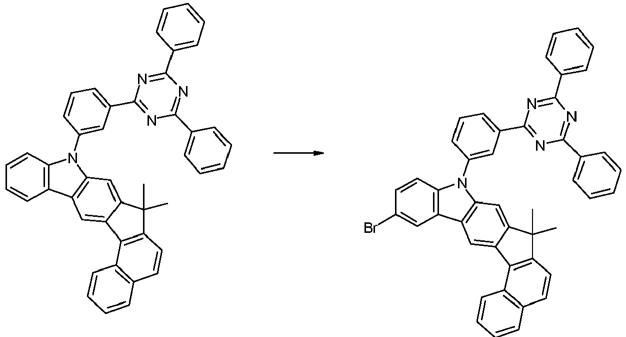

146 g (229 mmol) der Verbindung e werden in 1000 mL THF vorgelegt. Anschließend tropft man unter Lichtausschluss bei -15 °C eine Lösung aus 41.7 g (234.6 mmol) NBS in 500 mL THF zu, lässt auf Raumtemperatur kommen und rührt 4 h weiter bei dieser Temperatur. Anschließend wird die Mischung mit 150 mL Wasser versetzt und mit CH₂Cl₂ extrahiert. Die organische Phase wird über MgSO₄ getrocknet und die Lösungsmittel im Vakuum entfernt. Das Produkt wird mit Hexan heiß ausgerührt und abgesaugt. Ausbeute: 83 g (116 mmol); 51 % d. Th.; Reinheit nach ¹H-NMR ca. 98 %.

Analog können folgende Verbindungen hergestellt werden:

| | **Edukt** | **Produkt** | **Ausbeute** |
|---|---|---|---|
| 1h | 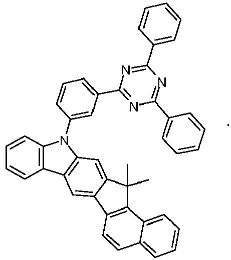 | 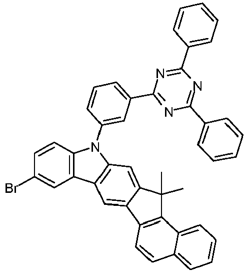 | 56% |

### i) Suzuki-Reaktion

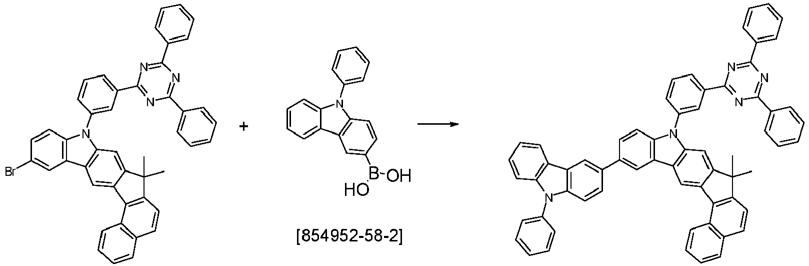

30.5 g (43 mmol ) der Verbindung aus Beispiel h, 13.4 g (47 mmol) 9-Phenylcarbazol-3-boronsäure und 29.2 g Rb₂CO₃ werden in 250 mL p-Xylol suspendiert. Zu dieser Suspension werden 0.95 g (4.2 mmol) Pd(OAc)₂ und 12.6 mL einer 1M Tri-tert-butylphosphinlösung in Toluol gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99.9 %. Ausbeute: 27 g (30 mmol); 72 % d. Th..

Analog können folgende Verbindungen hergestellt werden:

| | **Edukt** | **Produkt** | **Ausbeute** |
|---|---|---|---|
| 1i | 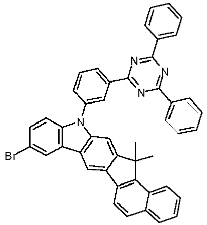 | 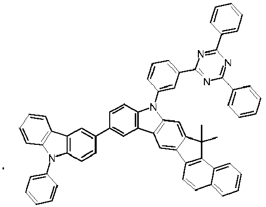 | 58% |

### Herstellung der OLEDs

In den folgenden Beispielen E1 bis E3 (siehe Tabelle 1) wird der Einsatz der erfindungsgemäßen Materialien in OLEDs vorgestellt.

**Vorbehandlung für die Beispiele E1-E3:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1: IC2:TER5 (55%:35%: 10%) bedeutet hierbei, dass das Material IC1 in einem Volumenanteil von 55%, IC2 in einem Anteil von 35% und TER5 in einem Anteil von 10% in der Schicht vorliegt. Weiterhin kann die Emissionsschicht auch aus mindestens einem Matrixmaterial und mehreren emittierenden Dotierstoffen, die dem Matrixmaterial bzw. den Matrixmaterialien durch Coverdampfung in einem bestimmten Volumenanteil beigemischt werden, bestehen. Dabei müssen nicht alle verwendeten Emitter zur Emission beitragen. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% in Tabelle 3 bedeutet, dass die in Spalte LD angegebene Lebensdauer der Zeit entspricht, nach der die Leuchtdichte auf 95% ihres Anfangswertes absinkt.

### Verwendung von erfindungsgemäßen Mischungen in OLEDs

Die erfindungsgemäße Verbindung EG1 wird in den Beispielen E1 bis E3 als Matrixmaterial in der Emissionsschicht phosphoreszierender roter OLEDs eingesetzt. Sowohl als Einzelmaterial (Beispiel E1) als auch in der Mischung mit einem lochleitenden Matrixmaterial (Beispiel E2) und in Kombination mit einem zweiten phosporeszierenden gelben Emitter (Beispiel E3) werden wesentliche Verbesserungen der Lebensdauer gegenüber dem Stand der Technik erreicht (V1 bis V3) bei ansonsten nahezu unveränderten Parametern. Als Stand der Technik wird eine Verbindung gemäß WO 2010/136109 eingesetzt.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 125nm | SpMA3 10nm | IC1:TER5 (97%:3%) 40nm | --- | ST2:LiQ (50%:50%) 35nm | --- |
| V2 | HATCN 5nm | SpMA1 125nm | SpMA3 10nm | IC1:IC2:TER5 (32%:65%:3%) 40nm | --- | ST2:LiQ (50%:50%) 35nm | --- |
| V3 | HATCN 5nm | SpMA4 135nm | --- | IC1 :TEY1 :TER6 (80%:15%:5%) 40nm | --- | ST2:LiQ (50%:50%) 35nm | --- |
| E1 | HATCN 5nm | SpMA1 125nm | SpMA3 10nm | EG1:TER5 (97%:3%) 40nm | --- | ST2:LiQ (50%:50%) 35nm | --- |
| E2 | HATCN 5nm | SpMA1 125nm | SpMA3 10nm | EG1:IC2:TER5 (32%:65%:3%) 40nm | --- | ST2:LiQ (50%:50%) 35nm | --- |
| E3 | HATCN 5nm | SpMA4 135nm | --- | EG1:TEY1:TER6 (80%:15%:5%) 40nm | --- | ST2:LiQ (50%:50%) 35nm | --- |

**Tabelle 2: Strukturformeln der Materialien für die OLEDs**

| | |
|---|---|
| 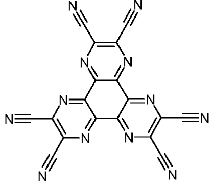 | 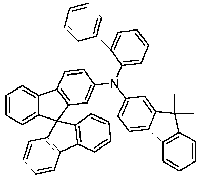 |
| HATCN | SpMA1 |
| 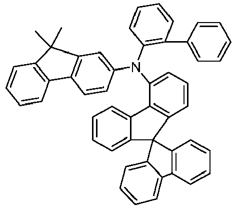 | 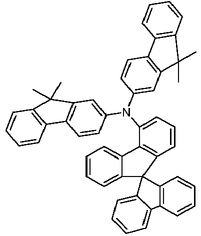 |
| SpMA3 | SpMA4 |
| 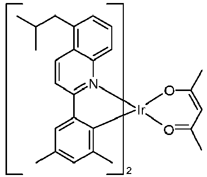 | 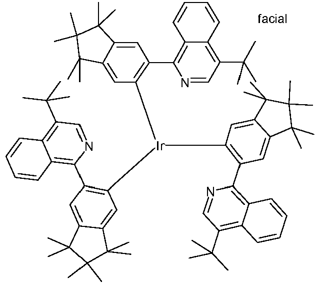 |
| TER5 | TER6 |
| 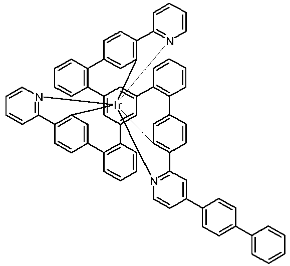 | 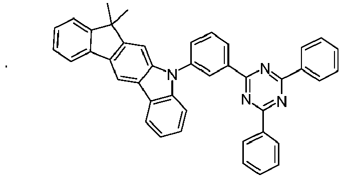 |
| TEY1 | IC1 |
| 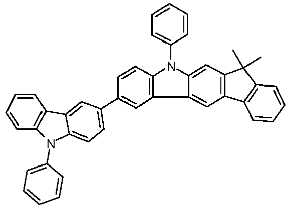 | 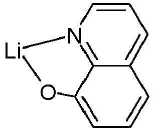 |
| IC2 | LiQ |
| 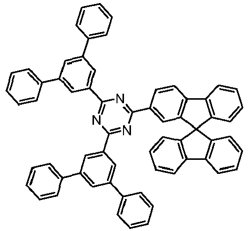 | 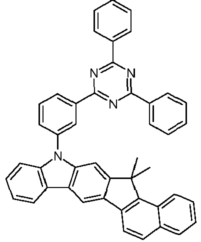 |
| ST2 | EG1 |

| Bsp. | CIE x/y bei 1000 cd/m² | j₀ (mA/cm²) | L1 (%) | LD (h) |
|---|---|---|---|---|
| V1 | 0.66/0.34 | 20 | 95 | 70 |
| V2 | 0.66/0.34 | 20 | 95 | 200 |
| V3 | 0.69/0.31 | 20 | 95 | 610 |
| E1 | 0.67/0.33 | 20 | 95 | 970 |
| E2 | 0.67/0.33 | 20 | 95 | 310 |
| E3 | 0.69/0.31 | 20 | 95 | 1420 |

## Patentansprüche

1. Verbindung gemäß Formel (1), 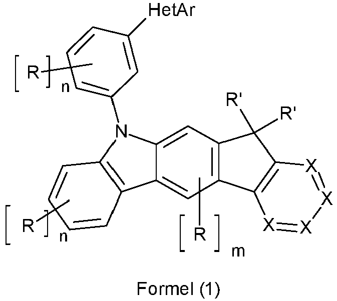 wobei für die verwendeten Symbole und Indizes gilt:
X zwei benachbarte X stehen für eine Gruppe der folgenden Formel (2), und die beiden anderen X stehen für CR, 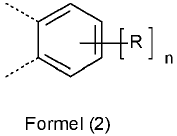 wobei die beiden gestrichelten Bindungen die Verknüpfung dieser Gruppe darstellen;
HetAr ist eine Gruppe gemäß einer der Formeln (HetAr-1), (HetAr-3), (HetAr-4) oder (HetAr-5), 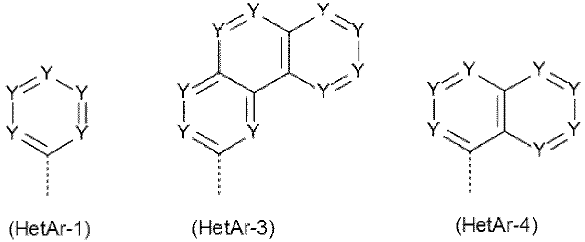 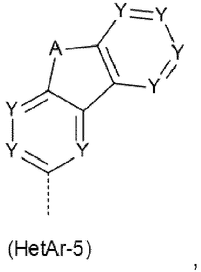 wobei die gestrichelte Bindung die Bindung an die Phenylengruppe darstellt;
Y ist gleich oder verschieden bei jedem Auftreten CR oder N, mit der Maßgabe, dass mindestens ein Symbol Y für N steht und dass maximal drei Symbole Y für N stehen;
A ist C(R¹)₂, NR¹, O oder S;
R ist bei jedem Auftreten gleich oder verschieden H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyloder Alkinylgruppe mit 2 bis 20 C-Atomen oder eine verzweigte oder cyclische Alkylgruppe mit 3 bis 20 C-Atomen, wobei die Alkyl, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches oder heteroaliphatisches Ringsystem bilden;
R' ist bei jedem Auftreten gleich oder verschieden eine geradkettige Alkylgruppe mit 1 bis 20 C-Atomen oder eine verzweigte oder cyclische Alkylgruppe mit 3 bis 20 C-Atomen, wobei die geradkettige, verzweigte oder cyclische Alkylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R' auch miteinander ein aromatisches, heteroaromatisches, aliphatisches oder heteroaliphatisches Ringsystem bilden;
Ar' ist bei jedem Auftreten gleich oder verschieden ein aromatisches oder heteroaromatisches Ringsystem mit 5 bis 40 aromatischen Ringatomen, welches mit einem oder mehreren Resten R¹ substituiert sein kann;
R¹ ist bei jedem Auftreten gleich oder verschieden H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 CAtomen, in dem auch ein oder mehrere H-Atome durch F ersetzt sein können;
m ist 0, 1 oder 2;
n ist bei jedem Auftreten gleich oder verschieden 0, 1, 2, 3 oder 4.

2. Verbindung nach Anspruch 1, ausgewählt aus den Verbindungen der Formeln (3a-1), (3a-2), (4a-1), (4a-2), (5a-1) und (5a-2), 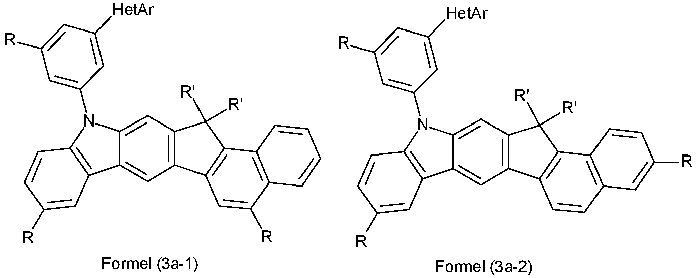 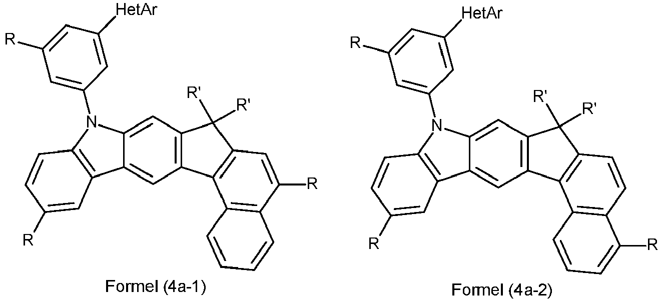 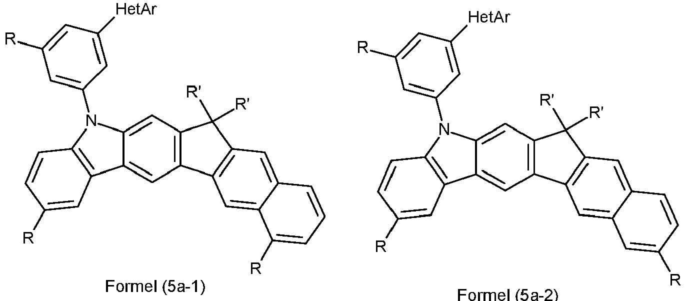 wobei HetAr, R und R' die in Anspruch 1 genannten Bedeutungen aufweisen.

3. Verbindung nach Anspruch 1 oder 2, ausgewählt aus den Verbindungen der Formeln (3b), (4b) und (5b), 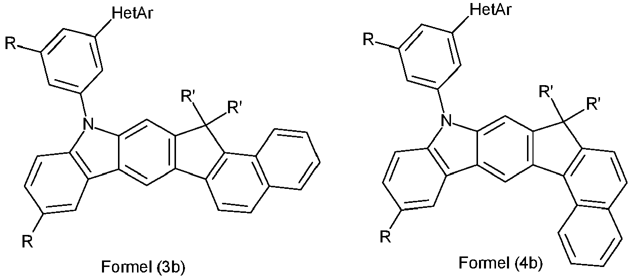 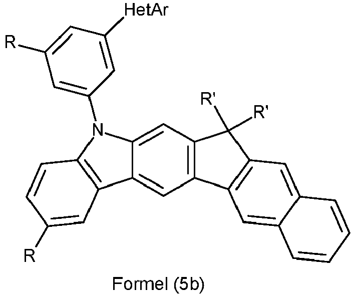 wobei HetAr, R und R' die in Anspruch 1 genannten Bedeutungen aufweisen.

4. Verbindung nach einem oder mehreren der Ansprüche 1 bis 3, ausgewählt aus den Verbindungen der Formeln (3c-1), (3c-2), (4c-1), (4c-2), (5c-1) und (5c-2), 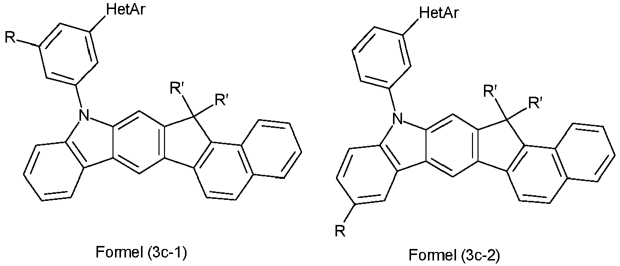 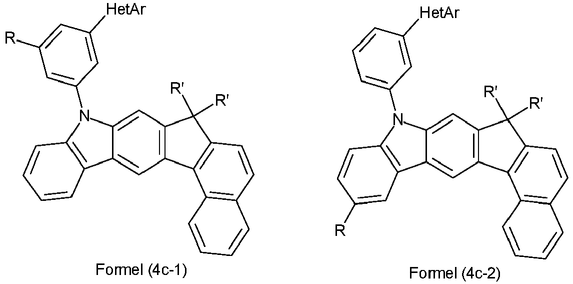 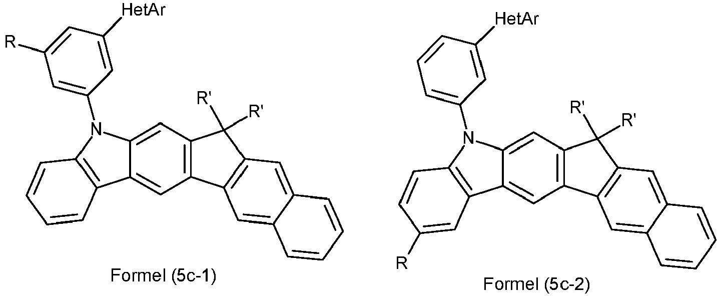 wobei HetAr, R und R' die in Anspruch 1 genannten Bedeutungen aufweisen.

5. Verbindung nach einem oder mehreren der Ansprüche 1 bis 4, **dadurch gekennzeichnet, dass** HetAr ausgewählt ist aus den Gruppen der Formeln (HetAr-1a) bis (HetAr-1d), (HetAr-3a), (HetAr-4a), (HetAr-5a) und (HetAr-5b), 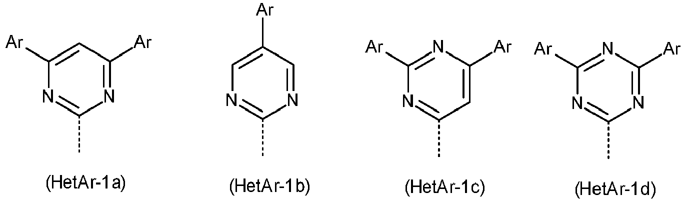 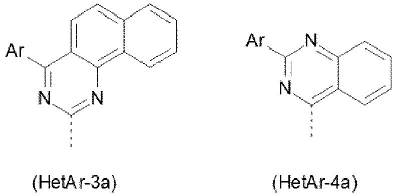 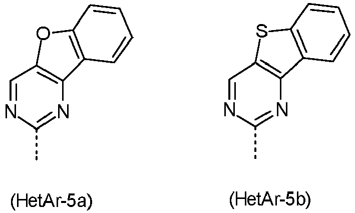 wobei die gestrichelte Bindung die Bindung an die Phenylengruppe darstellt, Ar gleich oder verschieden bei jedem Auftreten für ein aromatisches oder heteroaromatisches Ringsystem mit 5 bis 40 aromatischen Ringatomen steht, welches mit einem oder mehreren Resten R¹ substituiert sein kann, und R¹ die in Anspruch 1 genannten Bedeutungen aufweist.

6. Verbindung nach Anspruch 5, **dadurch gekennzeichnet, dass** Ar gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können.

7. Verbindung nach einem oder mehreren der Ansprüche 1 bis 6, **dadurch gekennzeichnet, dass** R gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂.

8. Verfahren zur Herstellung einer Verbindung nach einem oder mehreren der Ansprüche 1 bis 7, **dadurch gekennzeichnet, dass** das Grundgerüst synthetisiert wird, welches die Gruppe meta-Phenylen-HetAr noch nicht enthält, und dass die Gruppe meta-Phenylen-HetAr mittels einer nukleophilen aromatischen Substitutionsreaktion oder einer Kupplungsreaktion eingeführt wird.

9. Formulierung, enthaltend mindestens eine Verbindung nach einem oder mehreren der Ansprüche 1 bis 7 und mindestens eine weitere Verbindung, wobei die weitere Verbindung bevorzugt ausgewählt ist aus einem oder mehreren Lösemitteln, einer emittierenden Verbindung und/oder einem weiteren Matrixmaterial.

10. Verwendung einer Verbindung nach einem oder mehreren der Ansprüche 1 bis 7 in einer elektronischen Vorrichtung.

11. Elektronische Vorrichtung enthaltend mindestens eine Verbindung nach einem oder mehreren der Ansprüche 1 bis 7.

12. Elektronische Vorrichtung nach Anspruch 11, wobei es sich um eine organische Elektrolumineszenzvorrichtung handelt, **dadurch gekennzeichnet, dass** die Verbindung nach einem oder mehreren der Ansprüche 1 bis 7 als Matrixmaterial in einer emittierenden Schicht und/oder in einer Elektronentransportschicht und/oder in einer Lochblockierschicht eingesetzt werden.

13. Elektronische Vorrichtung nach Anspruch 12, **dadurch gekennzeichnet, dass** die Verbindung nach Anspruch 1 bis 7 als Matrixmaterial für phosphoreszierende Emitter eingesetzt wird in Kombination mit einem weiteren Matrixmaterial, welches ausgewählt ist aus Verbindungen gemäß einer der Formeln (6) oder (7), 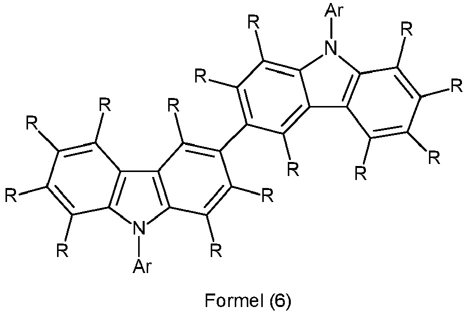 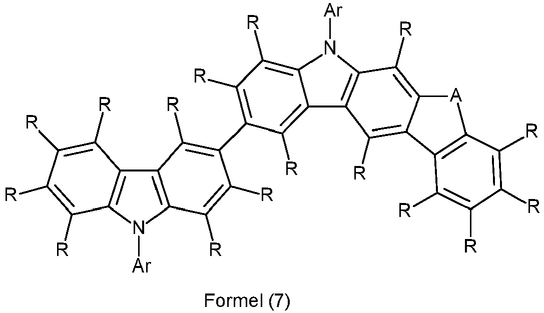 wobei Ar die in Anspruch 5 genannten Bedeutungen aufweist, R die in Anspruch 1 genannten Bedeutungen aufweist, jedoch Reste R hier auch miteinander ein aromatisches oder heteroaromatisches Ringsystem bilden können, und A für C(R')₂, NR', O oder S steht.

## Claims

1. Compound of formula (1) 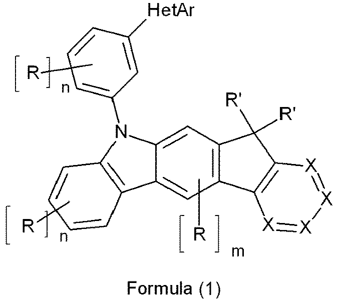 where the symbols and indices used are as follows:
X two adjacent X are a group of the formula (2) below, and the two other X are CR, 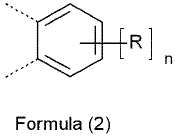 where the two dotted bonds represent the linkage of this group;
HetAr is a group of one of the formulae (HetAr-1), (HetAr-3), (HetAr-4) and (HetAr-5) 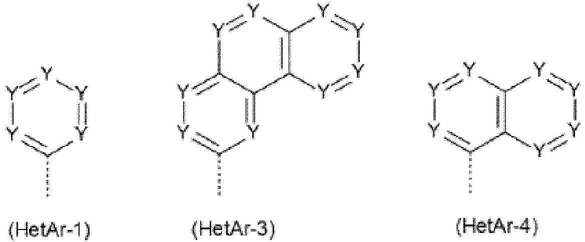 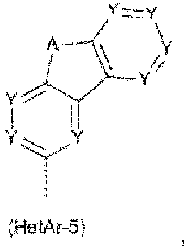 where the dotted bond represents the bond to the phenylene group;
Y is the same or different at each instance and is CR or N, with the proviso that at least one symbol Y is N and that not more than three symbols Y are N;
A is C(R¹)₂, NR¹, O or S;
R is the same or different at each instance and is H, D, F, Cl, Br, I, N(R¹)₂, N(Ar')₂, CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straightchain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals and where one or more nonadjacent CH₂ groups may be replaced by Si(R¹)₂, C=O, NR¹, O, S or CONR¹, or an aromatic or heteroaromatic ring system which has 5 to 60 aromatic ring atoms, preferably 5 to 40 aromatic ring atoms, and may be substituted in each case by one or more R¹ radicals; at the same time, two R radicals together may also form an aliphatic or heteroaliphatic ring system;
R' is the same or different at each instance and is a straight-chain alkyl group having 1 to 20 carbon atoms or a branched or cyclic alkyl group having 3 to 20 carbon atoms, where the straightchain, branched or cyclic alkyl group may in each case be substituted by one or more R¹ radicals and where one or more nonadjacent CH₂ groups may be replaced by O, or an aromatic or heteroaromatic ring system which has 5 to 40 aromatic ring atoms and may be substituted in each case by one or more R¹ radicals; at the same time, two R' radicals together may also form an aromatic, heteroaromatic, aliphatic or heteroaliphatic ring system;
Ar' is the same or different at each instance and is an aromatic or heteroaromatic ring system which has 5 to 40 aromatic ring atoms and may be substituted by one or more R¹ radicals;
R¹ is the same or different at each instance and is H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals, where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals; at the same time, two or more R¹ radicals together may form a ring system;
R² is the same or different at each instance and is H, D, F or an aliphatic, aromatic or heteroaromatic organic radical, especially a hydrocarbyl radical, having 1 to 20 carbon atoms, in which one or more hydrogen atoms may also be replaced by F;
m is 0, 1 or 2;
n is the same or different at each instance and is 0, 1, 2, 3 or 4.

2. Compound according to Claim 1, selected from the compounds of the formulae (3a-1), (3a-2), (4a-1), (4a-2), (5a-1) and (5a-2) 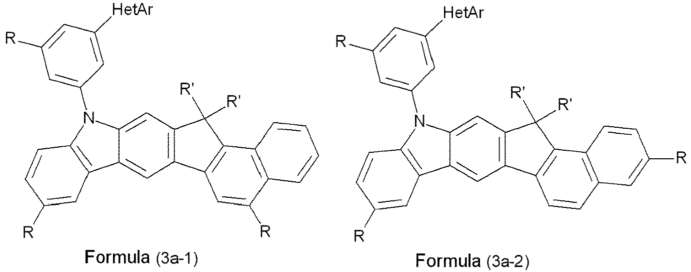 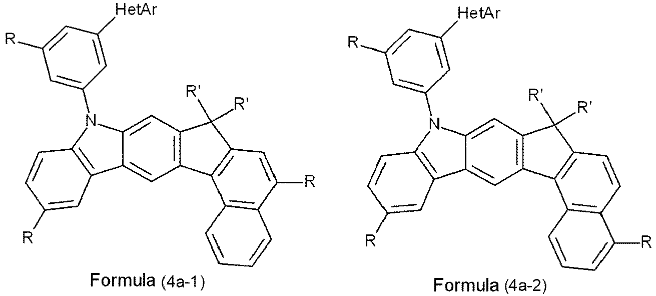 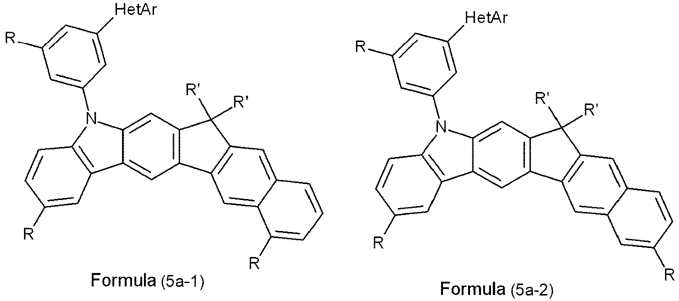 where HetAr, R and R' have the definitions given in Claim 1.

3. Compound according to Claim 1 or 2, selected from the compounds of the formulae (3b), (4b) and (5b) 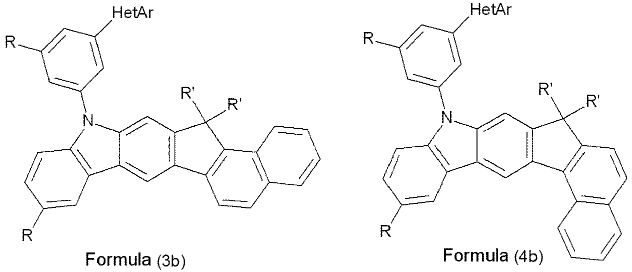 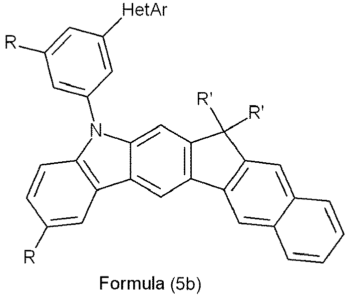 where HetAr, R and R' have the definitions given in Claim 1.

4. Compound according to one or more of Claims 1 to 3, selected from the compounds of the formulae (3c-1), (3c-2), (4c-1), (4c-2), (5c-1) and (5c-2) 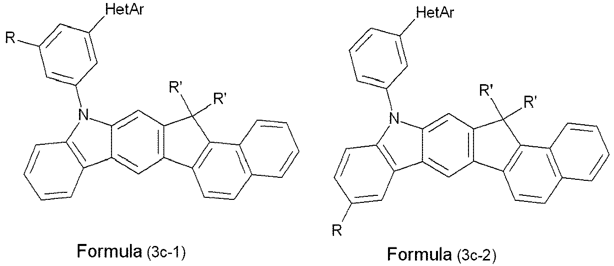 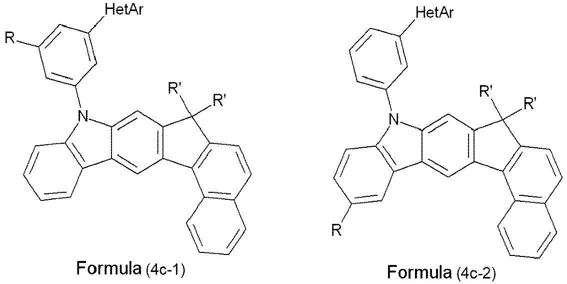 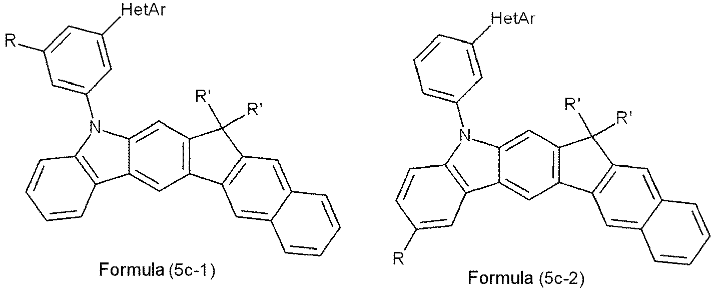 where HetAr, R and R' have the definitions given in Claim 1.

5. Compound according to one or more of Claims 1 to 4, **characterized in that** HetAr is selected from the groups of the formulae (HetAr-1a) to (HetAr-1d), (HetAr-3a), (HetAr-4a), (HetAr-5a) and (HetAr-5b) 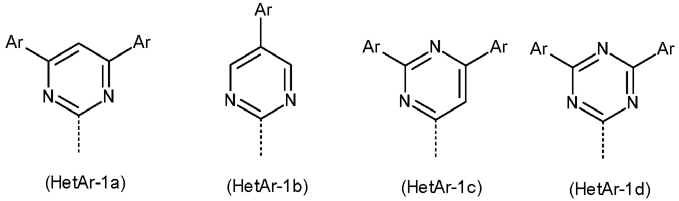 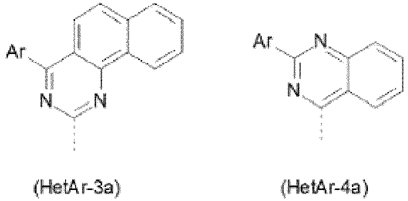 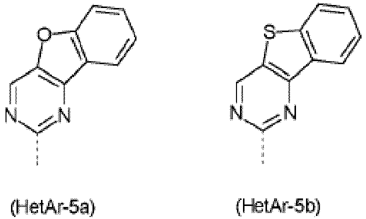 where the dotted bond represents the bond to the phenylene group, Ar is the same or different at each instance and is an aromatic or heteroaromatic ring system which has 5 to 40 aromatic ring atoms and may be substituted by one or more R¹ radicals, and R¹ has the definitions given in Claim 1.

6. Compound according to Claim 5, **characterized in that** Ar is the same or different at each instance and is selected from phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, quinoline, isoquinoline, quinazoline, quinoxaline, phenanthrene and triphenylene, each of which may be substituted by one or more R¹ radicals.

7. Compound according to one or more of Claims 1 to 6, **characterized in that** R is the same or different at each instance and is selected from the group consisting of H, D, an aromatic or heteroaromatic ring system which has 6 to 30 aromatic ring atoms and may be substituted by one or more R¹ radicals, and an N(Ar')₂ group.

8. Process for preparing a compound according to one or more of Claims 1 to 7, **characterized in that** the base skeleton that does not as yet contain the meta-phenylene-HetAr group is synthesized, and **in that** the meta-phenylene-HetAr group is introduced by means of a nucleophilic aromatic substitution reaction or a coupling reaction.

9. Formulation comprising at least one compound according to one or more of Claims 1 to 7 and at least one further compound, wherein the further compound is preferably selected from one or more solvents, an emitting compound and/or a further matrix material.

10. Use of a compound according to one or more of Claims 1 to 7 in an electronic device.

11. Electronic device comprising at least one compound according to one or more of Claims 1 to 7.

12. Electronic device according to Claim 11 which is an organic electroluminescent device, **characterized in that** the compound according to one or more of Claims 1 to 7 is used as matrix material in an emitting layer and/or in an electron transport layer and/or in a hole blocker layer.

13. Electronic device according to Claim 12, **characterized in that** the compound according to Claims 1 to 7 is used as matrix material for phosphorescent emitters in combination with a further matrix material selected from compounds of one of the formulae (6) and (7) 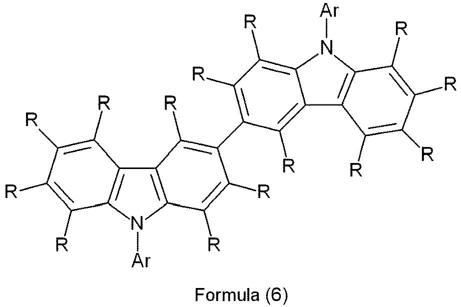 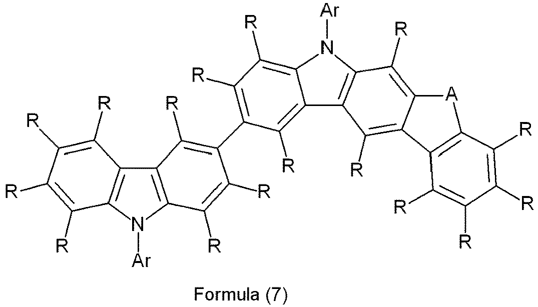 where Ar has the definitions given in Claim 5, R has the definitions given in Claim 1, but R radicals here may also together form an aromatic or heteroaromatic ring system, and A is C(R')₂, NR', O or S.

## Revendications

1. Composé selon la formule (1) 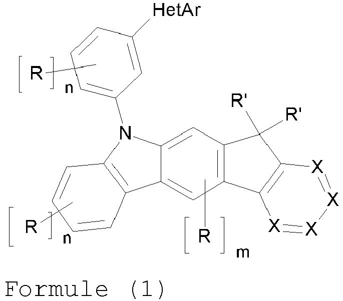 où, pour les symboles et indices utilisés, ce qui suit est d'application :
X deux X adjacents représentent un groupe de la formule (2) suivante et les deux autres X représentent CR, 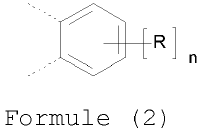 les deux liaisons en pointillés représentant la liaison de ce groupe ;
HetAr représente un groupe selon l'une des formules (HetAr-1), (HetAr-3), (HetAr-4) ou (HetAr-5), 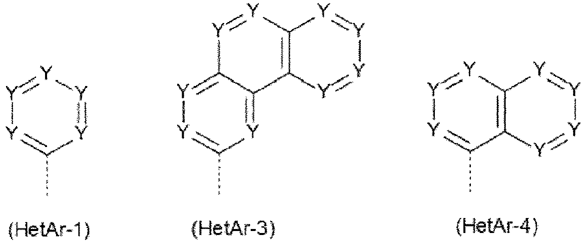 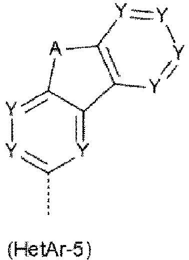 la liaison en pointillés représentant la liaison au groupe phénylène ;
Y représente, en chaque occurrence, de manière identique ou différente, CR ou N, sous réserve qu'au moins un symbole Y représente N et qu'au maximum trois symboles Y représentent N ;
A représente C(R¹)₂, NR¹, O ou S ;
R représente, en chaque occurrence, de manière identique ou différente, H, D, F, Cl, Br, I, N(R¹)₂, N(Ar')₂, CN, NO₂, OR¹, SR¹, COOR¹, C(=O)N(R¹)₂, Si(R¹)₃, B(OR¹⁾₂, C(=O)R¹, P(=O)(R¹)₂, S(=O)R¹, S(=O)₂R¹, OSO₂R¹, un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R¹ et un ou plusieurs groupes CH₂ non adjacents pouvant être remplacés par Si(R¹)₂, C=O, NR¹, O, S ou CONR¹, ou un système cyclique aromatique ou hétéroaromatique comprenant 5 à 60 atomes de cycle aromatique, de préférence 5 à 40 atomes de cycle aromatique, qui peut à chaque fois être substitué par un ou plusieurs radicaux R¹ ; deux radicaux R pouvant également former ensemble un système cyclique aliphatique ou hétéroaliphatique ;
R' représente, en chaque occurrence, de manière identique ou différente, un groupe alkyle linéaire comprenant 1 à 20 atomes de carbone ou un groupe alkyle ramifié ou cyclique comprenant 3 à 20 atomes de carbone, le groupe alkyle linéaire, ramifié ou cyclique pouvant à chaque fois être substitué par un ou plusieurs radicaux R¹ et un ou plusieurs groupes CH₂ non adjacents pouvant être remplacés par O, ou un système cyclique aromatique ou hétéroaromatique comprenant 5 à 40 atomes de cycle aromatique, qui peut à chaque fois être substitué par un ou plusieurs radicaux R¹ ; deux radicaux R' pouvant également former ensemble un système cyclique aromatique, hétéroaromatique, aliphatique ou hétéroaliphatique ;
Ar' représente, en chaque occurrence, de manière identique ou différente, un système cyclique aromatique ou hétéroaromatique comprenant 5 à 40 atomes de cycle aromatique, qui peut être substitué par un ou plusieurs radicaux R¹ ;
R¹ représente, en chaque occurrence, de manière identique ou différente, H, D, F, Cl, Br, I, N(R²)₂, CN, NO₂, OR², SR², Si(R²)₃, B(OR²)₂, C(=O)R², P(=O)(R²)₂, S(=O)R², S(=O)₂R², OSO₂R², un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R², un ou plusieurs groupes CH₂ non adjacents pouvant être remplacés par Si(R²)₂, C=O, NR², O, S ou CONR², ou un système cyclique aromatique ou hétéroaromatique comprenant 5 à 40 atomes de cycle aromatique, qui peut à chaque fois être substitué par un ou plusieurs radicaux R² ; deux radicaux R¹ ou plus pouvant former ensemble un système cyclique ;
R² représente, en chaque occurrence, de manière identique ou différente, H, D, F ou un radical organique, en particulier un radical hydrocarboné, aliphatique, aromatique ou hétéroaromatique, comprenant 1 à 20 atomes de carbone, dans lequel un ou plusieurs atomes H peuvent également être remplacés par F ;
m vaut 0, 1 ou 2 ;
n représente, en chaque occurrence, de manière identique ou différente, 0, 1, 2, 3 ou 4.

2. Composé selon la revendication 1, choisi parmi les composés des formules (3a-1), (3a-2), (4a-1), (4a-2), (5a-1) et (5a-2), 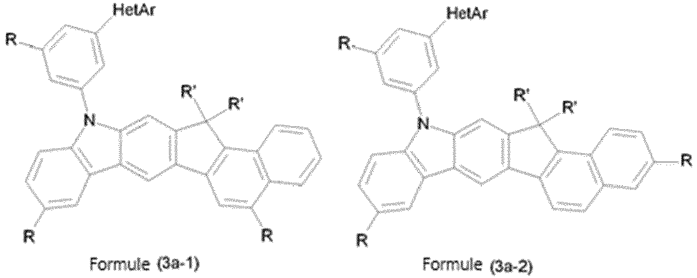 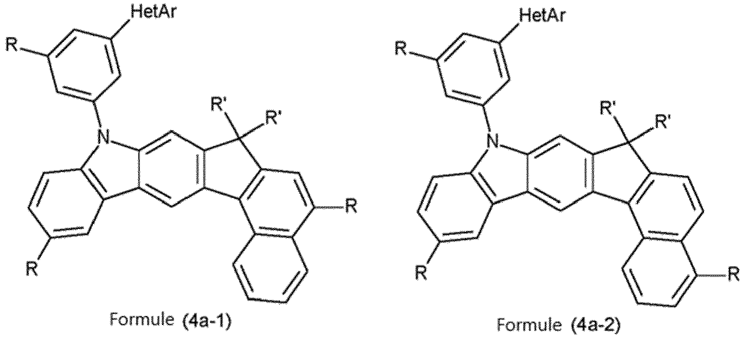 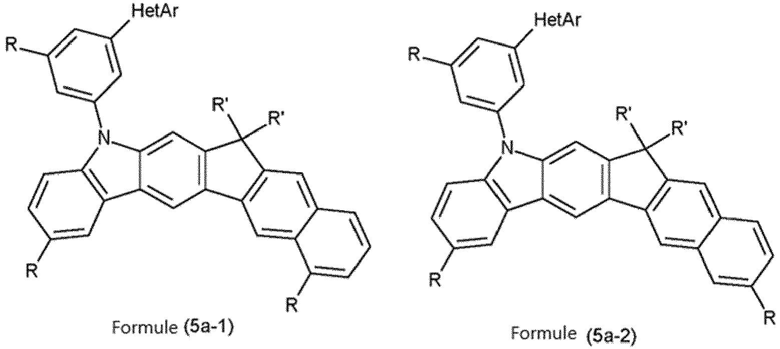 dans lesquelles HetAr, R et R' présentent les significations mentionnées dans la revendication 1.

3. Composé selon la revendication 1 ou 2, choisi parmi les composés des formules (3b), (4b) et (5b), 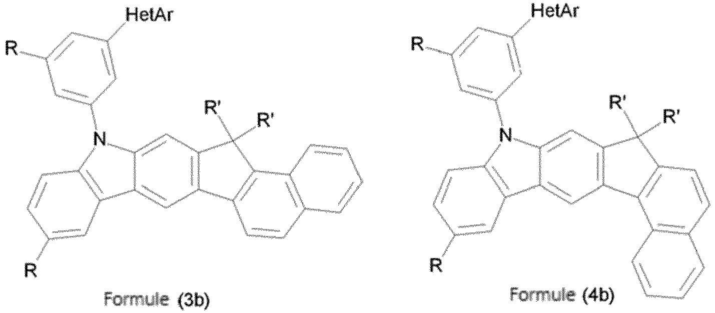 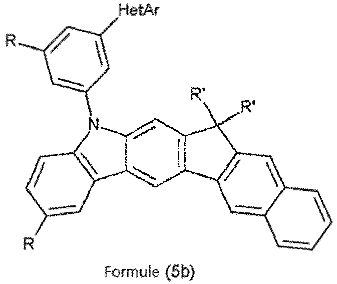 dans lesquelles HetAr, R et R' présentent les significations mentionnées dans la revendication 1.

4. Composé selon l'une ou plusieurs des revendications 1 à 3, choisi parmi les composés des formules (3c-1), (3c-2), (4c-1), (4c-2), (5c-1) et (5c-2), 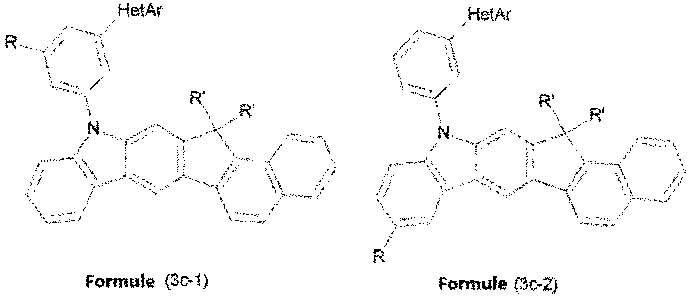 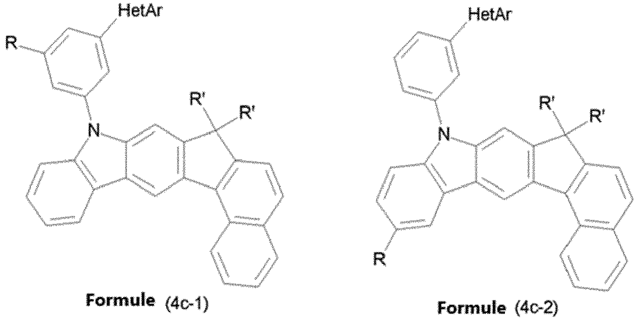 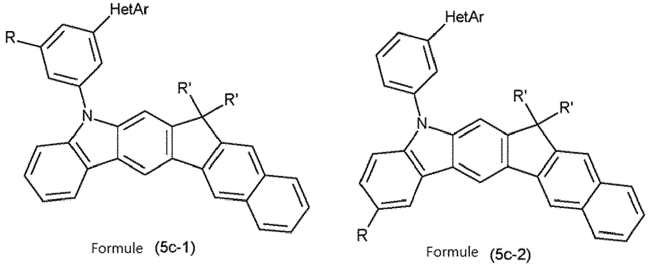 dans lesquelles HetAr, R et R' présentent les significations mentionnées dans la revendication 1.

5. Composé selon l'une ou plusieurs des revendications 1 à 4, **caractérisé en ce que** HetAr est choisi parmi les groupes des formules (HetAr-1a) à (HetAr-1d), (HetAr-3a), (HetAr-4a), (HetAr-5a) et (HetAr-5b), 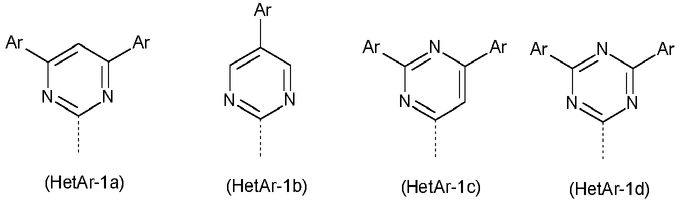 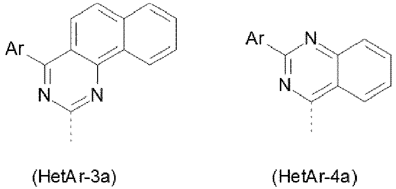 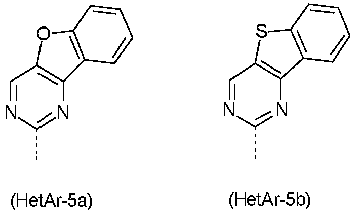 dans lesquelles la liaison en pointillés représente la liaison au groupe phénylène, Ar représente, en chaque occurrence, de manière identique ou différente, un système cyclique aromatique ou hétéroaromatique comprenant 5 à 40 atomes de cycle aromatique, qui peut être substitué par un ou plusieurs radicaux R¹ et R¹ présente les significations mentionnées dans la revendication 1.

6. Composé selon la revendication 5, **caractérisé en ce que** Ar est choisi, en chaque occurrence, de manière identique ou différente, parmi phényle, biphényle, terphényle, quaterphényle, fluorène, spirobifluorène, naphtalène, indole, benzofuranne, benzothiophène, carbazole, dibenzofuranne, dibenzothiophène, indénocarbazole, indolocarbazole, pyridine, pyrimidine, pyrazine, pyridazine, triazine, quinoléine, isoquinoléine, quinazoline, quinoxaline, phénanthrène ou triphénylène, qui peuvent à chaque fois être substitués par un ou plusieurs radicaux R¹.

7. Composé selon l'une ou plusieurs des revendications 1 à 6, **caractérisé en ce que** R est choisi, en chaque occurrence, de manière identique ou différente, dans le groupe constitué par H, D, un système cyclique aromatique ou hétéroaromatique comprenant 6 à 30 atomes de cycle aromatique, qui peut être substitué par un ou plusieurs radicaux R¹, ou un groupe N(Ar')₂.

8. Procédé pour la préparation d'un composé selon l'une ou plusieurs des revendications 1 à 7, **caractérisé en ce que** la structure de base, qui ne contient pas encore le groupe méta-phénylène-HetAr, est synthétisée et **en ce que** le groupe méta-phénylène-HetAr est introduit au moyen d'une réaction de substitution aromatique nucléophile ou d'une réaction de couplage.

9. Formulation, contenant au moins un composé selon l'une ou plusieurs des revendications 1 à 7 et au moins un autre composé, l'autre composé étant de préférence choisi parmi un ou plusieurs solvants, un composé émetteur et/ou un autre matériau de matrice.

10. Utilisation d'un composé selon l'une ou plusieurs des revendications 1 à 7 dans un dispositif électronique.

11. Dispositif électronique contenant au moins un composé selon l'une ou plusieurs des revendications 1 à 7.

12. Dispositif électronique selon la revendication 11, le dispositif étant un dispositif organique électroluminescent, **caractérisé en ce que** le composé selon l'une ou plusieurs des revendications 1 à 7 est utilisé comme matériau de matrice dans une couche émettrice et/ou dans une couche de transport d'électrons et/ou dans une couche de blocage de trous.

13. Dispositif électronique selon la revendication 12, **caractérisé en ce que** le composé selon la revendication 1 à 7 est utilisé comme matériau de matrice pour des émetteurs phosphorescents en combinaison avec un autre matériau de matrice qui est choisi parmi les composés selon l'une des formules (6) ou (7), 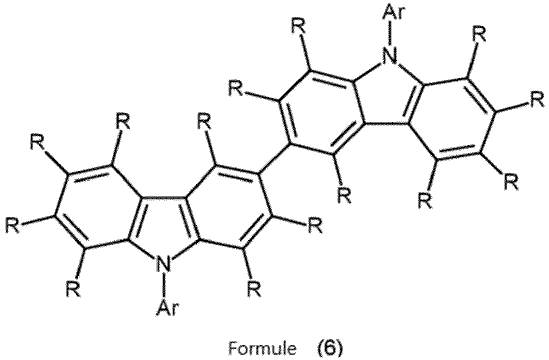 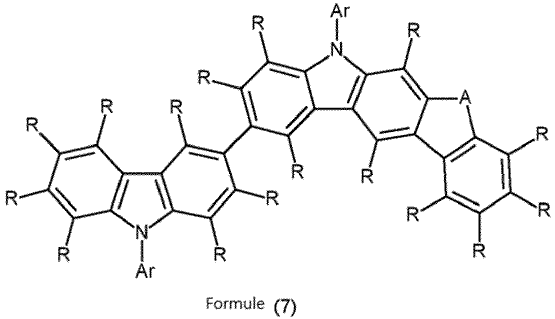 dans lesquelles Ar présente les significations mentionnées dans la revendication 5, R présente les significations mentionnées dans la revendication 1, mais les radicaux R peuvent ici également former ensemble un système cyclique aromatique ou hétéroaromatique, et A représente C(R')₂, NR', O ou S.
